(19) Europäisches Patentamt
European Patent Office
Office européen des brevets

(11) **EP 3 280 512 B1**

(12) **EUROPÄISCHE PATENTSCHRIFT**

(45) Veröffentlichungstag und Bekanntmachung des Hinweises auf die Patenterteilung:
**16.10.2024   Patentblatt 2024/42**

(21) Anmeldenummer: **16725027.3**

(22) Anmeldetag: **07.04.2016**

(51) Internationale Patentklassifikation (IPC):
*B01D 53/04* (2006.01)   *C07C 29/151* (2006.01)
*C10G 2/00* (2006.01)   *C25B 1/04* (2021.01)
*B01D 53/047* (2006.01)   *C25B 15/02* (2021.01)
*C25B 15/08* (2006.01)

(52) Gemeinsame Patentklassifikation (CPC):
*C10G 2/50; B01D 53/0462; B01D 53/047;*
*C25B 1/04; C25B 15/02; C25B 15/08;*
B01D 2257/504; B01D 2259/65; Y02C 20/20;
Y02C 20/40; Y02E 60/36; Y02P 20/129;
Y02P 20/151

(86) Internationale Anmeldenummer:
**PCT/DE2016/100164**

(87) Internationale Veröffentlichungsnummer:
**WO 2016/162022 (13.10.2016 Gazette 2016/41)**

(54) **HERSTELLUNGSVERFAHREN SOWIE HERSTELLUNGSANLAGE ZUR HERSTELLUNG VON METHAN / GASFÖRMIGEN UND/ODER FLÜSSIGEN KOHLENWASSERSTOFFEN**

PRODUCTION PROCESS AND PRODUCTION SYSTEM FOR PRODUCING METHANE / GASEOUS AND/OR LIQUID HYDROCARBONS

PROCÉDÉ ET INSTALLATION POUR LA PRODUCTION DE MÉTHANE/D'HYDROCARBURES GAZEUX ET/OU LIQUIDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.04.2015   PCT/DE2015/100148**

(43) Veröffentlichungstag der Anmeldung:
**14.02.2018   Patentblatt 2018/07**

(73) Patentinhaber:
• **Sunfire GmbH**
  **01237 Dresden (DE)**
• **Climeworks AG**
  **8050 Zürich (CH)**

(72) Erfinder:
• **VON OLSHAUSEN, Christian**
  **01324 Dresden (DE)**

• **RÜGER, Dietmar**
  **01728 Bannewitz (DE)**
• **WURZBACHER, Jan, Andre**
  **8037 Zürich (CH)**
• **GEBALD, Christoph**
  **8105 Regensdorf (CH)**

(74) Vertreter: **Hauck Patentanwaltspartnerschaft mbB**
  **Postfach 11 31 53**
  **20431 Hamburg (DE)**

(56) Entgegenhaltungen:
**EP-A1- 2 491 998**          **EP-A1- 2 638 949**
**DE-A1- 102007 056 267**    **US-A1- 2014 272 734**

**EP 3 280 512 B1**

**Beschreibung**

Die Erfindung betrifft ein Herstellungsverfahren von synthetisch hergestelltem Methan / gasförmigen und/oder flüssigen Kohlenwasserstoffen, wobei hierzu Wasserstoff aus einer mit Hilfe von regenerativ erzeugter Elektroenergie betriebenen Elektrolyseanordnung und Kohlenstoffdioxid in einer Methan- oder Fischer-Tropsch-Synthese oder anderen geeigneten Kohlenwasserstoffsynthese kombiniert werden sowie eine Herstellungsanlage zur Produktion von synthetisch hergestelltem Methan / gasförmigen und/oder flüssigen Kohlenwasserstoffen.

Aus dem **Stand der Technik** sind unterschiedliche Anordnungen bekannt, um Methan oder gasförmige bzw. flüssige Kohlenwasserstoffe unter Einbeziehung von Elektroenergie synthetisch herzustellen. Hierbei dienen derartige Syntheseanlagen vor allem der Speicherung von elektrischer Energie, die auf regenerative Weise, bspw. durch Wind- oder Sonnenenergie, hergestellt wurde und stellen Stromspeicher dar. Weiter dienen derartige Syntheseanlagen der Herstellung von synthetisch hergestellten und $CO_2$-neutralen Kraftstoffen allgemein. Exemplarisch sei hier auf die Druckschrift EP 2 049 232 B der Anmelderin hingewiesen, aus der ein Verfahren zur Wiederaufarbeitung der Verbrennungsprodukte Kohlendioxid und Wasser in erneuerbare energetische Brenn- und Kraftstoffe mithilfe elektrischer Energie aus erneuerbaren Energiequellen bekannt ist.

Ferner ist aus der Druckschrift EP 1 887 071 A1 ein Synthesegesamtverfahren bekannt, wobei zur Herstellung von Synthesegas Kohlendioxid aus Prozessabgasen oder der Normalatmosphäre entnommen werden kann, wobei zur Entnahme das sog. Oxyfuel-Verfahren oder aber bei der Kraftwerkstechnik nachgeschaltete $CO_2$-Abgasstromabscheidanlagen vorgeschlagen werden. Besonders bevorzugt werden jedoch zur $CO_2$-Gewinnung Luftzerlegungsanlagen angeführt, die für die Erzielung von Stickstoff und/oder Sauerstoff im Einsatz sind. Ferner werden noch $CO_2$-Abscheideverfahren mit Einsatz von gradierten Membranen weiter angeführt.

Aus der Druckschrift WO 2008/021698 A2 ist ein Verfahren zur Herstellung von Methan und anderen Produkten bekannt, wobei hierzu das notwendige Kohlendioxid durch Absorption oder Adsorption von getrockneter atmosphärischer Luft erhalten wird.

Weiter ist aus der Druckschrift WO 2006/004583 A2 bekannt, dass das zur Herstellung von synthetischen flüssigen Kohlenwasserstoffen benötigte Kohlendioxid aus Seewasser oder der Umgebungsluft entnommen werden kann.

Aus der Druckschrift GB 2,448,685 ist ein Verfahren bzw. eine Anordnung bekannt, bei der zur Synthetisierung von flüssigen Kohlenwasserstoffen bzw. Kraftstoffen Kohlendioxid, gewonnen aus der Luft durch Absorption oder Adsorption, verwendet wird.

Die Druckschrift US 2014/0272734 A1 beschreibt Prozesse zur Herstellung von flüssigen Kraftstoffen aus den Ausgangsstoffen $CO_2$ und $H_2O$. In Bezug auf $CO_2$ Quellen beschreibt diese Druckschrift ausschliesslich Verbrennungsabgase ("flue gas") [0014, 0027], wohingegen atmosphärische Luft als $CO_2$ Quelle keinerlei Erwähnung findet. Im Unterschied zu dieser Druckschrift wird in der diesseitigen Offenbarung atmosphärische Luft als $CO_2$ Quelle verwendet. Aufgrund der hohen Verdünnung von $CO_2$ in atmosphärischer Luft unterscheiden sich Apparate und zugehörige Prozesse zur $CO_2$ Abscheidung aus Luft und aus Rauchgasen grundsätzlich. An dieser Stelle soll angemerkt werden, dass ein Rauchgas ca. 300 mal mehr $CO_2$ enthält als in der atmosphärischen Luft enthalten ist. Der in der vorliegenden Offenbarung beschriebene Prozess zur $CO_2$ Abscheidung aus Luft basiert auf einem Temperatur-Vakuum-Wechselverfahren für Amin-funktionalisierte Adsorbenzien. Aufgrund der niedrigen $CO_2$ Konzentration von $CO_2$ in der Luft ist im Speziellen eine Druckreduktion und Temperaturerhöhung für eine effiziente und schonende Regeneration des Adsorbens notwendig. Die Druckschrift US 2014/0272734 A1 beschreibt ausschliesslich Druckwechsel-(pressure-swing) und Temperaturwechselverfahren (temperature-swing adsorption) [0027] als mögliche Verfahren für Adsorbenzien. Jedoch reicht ein Druckwechsel alleine für eine bewusste $CO_2$ Abscheidung aus der Luft nicht aus. Für die Regeneration würde ein Temperaturwechsel alleine zu einer Schädigung des Adsorbens führen. Ein Hauptunterschied in der Prozessführung zwischen der Druckschrift US 2014/0272734 A1 und der diesseitigen Offenbarung ist die Co-Adsorption und Co-Desorption von $H_2O$. Die Konzentration von $H_2O$ in atmosphärischer Luft ist meistens eine Grössenordnung grösser als die Konzentration von $CO_2$, wohingegen in Rauchgasen die Konzentration von $H_2O$ maximal so gross ist wie die Konzentration von $CO_2$, meistens jedoch deutlich geringer. Aus diesem Grund wird für $CO_2$ Abscheidung aus Luft ein signifikanter Teil $H_2O$ co-adsorbiert, welches während der $CO_2$ Desorption co-desorbiert wird. Die Desorption von $H_2O$ hat eine fundamental andere Charakteristik als die Desorption von $CO_2$, weshalb die Methoden aus der Druckschrift US 2014/0272734 A1 keine Hinweise auf einen funktionierenden Prozess zur $CO_2$ Abscheidung aus Luft ermöglichen können, insbesondere nicht in Bezug auf eine Wärmeintegration. Zuletzt erwähnt die Druckschrift US 2014/0272734 A1 zwar, dass der Eingangsstrom $CO_2$ und $H_2O$ in die SOEC mit Druckluft vermischt werden kann, jedoch ist die Druckluft hierbei keine $CO_2$ Quelle sondern der Reduktionsreaktion in der SOEC dienlich bzw. geschuldet. Ferner wird im Patent US 2014/0272734 A1 erwähnt, dass vorgewärmte Luft der Elektrolyse zugeführt wird und diese mit dem vorgewärmten Feedstrom zu einem Syngas reagiert, wobei diese Vorstellung vermutlich aus dem Betrieb der SOFC stammt, wo die Luft an der Reaktion teilnimmt. Für den Betrieb einer SOEC, bzw. Co-Elektrolyse ist derartiges Vorgehen ohne Bedeutung. Die Luft dient lediglich dazu, den in der Elektrolyse durch Elektroenergie aus $H_2O$ und $CO_2$ abgespalteten Sauerstoff,

der sich auf der Anodenseite ansammelt, wegzuspülen. Hauptsächlich der $N_2$-Gehalt in der Luft reduziert dabei den Partialdruck von Sauerstoff auf der Anodenseite, was die Triebkraft für den Sauerstofftransport durch den Elektrolyt erhöht. Das in der Luft enthaltende $CO_2$ bleibt dabei unberührt, da es sich auf der Anodenseite und nicht wie der Feedstrom auf der Kathodenseite befindet.

Die Druckschrift EP 2 638 949 A1 beschreibt eine Vorrichtung und einen Prozess zur $CO_2$ Abscheidung aus Prozessgasströmen, insbesondere während der Methanol- oder Ammoniakproduktion. Der Prozess basiert auf Druck- und Temperaturwechselverfahren für einen Adsorbens ohne chemische Aminmodifizierung. Adsorbenzien ohne chemische Aminmodifizierung, z.B. Zeolithen, eignen sich nicht für die $CO_2$ Abscheidung aus Luft, da diese $CO_2$ und $H_2O$ kompetitiv adsorbieren. Aufgrund der vergleichsweisen grossen $H_2O$ Konzentration in Luft adsorbieren Adsorbenzien ohne chemische Aminmodifizierung bei Verwendung in atmosphärischer Luft hauptsächlich $H_2O$ (> 10mmol $H_2O$/g) und kaum $CO_2$ (< 0.1 mmol $CO_2$/g). Adsorbenzien ohne chemische Aminmodifizierung werden anschliessend bei vergleichsweise hohen Temperaturen von 200°C regeneriert, wie in der Druckschrift EP 2 638 949 A1 beschrieben [0014]. Amin- funktionalisierte Adsorbenzien, wie in der diesseitigen Offenbarung zur $CO_2$ Abscheidung aus Luft beschrieben, funktionieren fundamental anders als in der EP 2 638 949 A1 beschrieben: erstens adsorbiert diese Klasse Adsorbenzien $CO_2$ in der Präsenz von Wasser (keine kompetitiven Effekte) und zweitens werden diese bei deutlich geringeren Temperaturen von ca. 60-100°C regeneriert. Die Methoden aus EP 2 638 949 A1 lassen keine Rückschlüsse auf einen funktionierenden Prozess zur $CO_2$ Abscheidung aus Luft zu, inbesondere nicht in Bezug auf Wärmeintegration.

In der im Stand der Technik bekannten Druckschrift EP 2 491 998 A1 wird erwähnt, dass $CO_2$ aus Abgasen von Verbrennungsprozessen oder aus der Umgebung stammen kann. Eine, wie diesseits wesentliche $CO_2$-Gewinnungs- anlage, ist der Druckschrift EP 2 491 998 A1 nicht zu entnehmen, wodurch ein entscheidendes Merkmal fehlt. In der Druckschrift EP 2 491 998 A1 wird vorgeschlagen, das im Prozess anfallende Wasser zusammen mit Zusatzwasser zur Prozesskühlung im Bereich der Synthese einzusetzen und den dabei gebildeten Dampf in einer Elektrolyse mit Elektroenergie zu Wasserstoff und Sauerstoff aufzuspalten. Der Wasserstoff wird dann ebenfalls mit Hilfe von Elektroenergie zur Reduktion des $CO_2$ zu CO eingesetzt. Das erzeugte Synthesegas dient in einer Synthese zur Herstellung von Kohlenwasserstoffen. Somit besteht der Hauptunterschied der diesseitigen Offenbarung zur Druckschrift EP 2 491 998 A1 darin, dass der in der Druckschrift EP 2 491 998 A1 aus der Abwärme des Syntheseprozesses erzeugte Wasserdampf in einer Elektrolyse zur Erzeugung von Wasserstoff und nicht zur Wärmebereitstellung für einen $CO_2$-Gewinnungspro- zess genutzt wird.

Die Probleme im Stand der Technik sind im Wesentlichen die Ineffizienz von gekoppelten Syntheseanlagen in Verbindung mit Kohlendioxidherstellungsanlagen im Sinne der Erfindung, so dass hier neben dem allgemeinen Bestreben nach Verbesserung insbesondere die Energieeffizienzsteigerung von großer Bedeutung ist, wobei Ressourcen geschont werden sollen und der Aspekt der Neutralität in Bezug auf Energieverwendung im Vordergrund steht.

Es wurde insbesondere erkannt, dass bei im Stand der Technik befindlichen Syntheseanlagen ein Großteil noch nicht genutzter Abwärme vorliegt, deren Energie nutzbar gemacht werden soll.

Weiter wurde festgestellt, dass der Stand der Technik das Problem der schwankend anfallenden regenerativen Energie nicht löst.

Der vorliegenden Erfindung liegt die Aufgabe zugrunde, ein Herstellungsverfahren und eine Herstellungsanlage aufzuzeigen, die ein ressourcenschonendes und effizientes Herstellen von synthetisch hergestelltem Methan bzw. gasförmigen und/oder flüssigen Kohlenwasserstoffen bevorzugt aus schwankend anfallender Energie ermöglichen, wobei sich ergebende Nachteile der Einzelanlagenteile synergetisch miteinander zur Gesamtanlage verbunden werden sollen und insbesondere nutzbare Abwärme der Syntheseanlage genutzt werden soll.

Gelöst wird diese Aufgabe mit einem Herstellungsverfahren gemäß Hauptanspruch sowie einer Herstellungsanlage gemäß nebengeordnetem Anspruch.

Das Kohlenstoffdioxid wird aus einem Luftstrom mittels einer Kohlenstoffdioxidgewinnungsanlage hergestellt, wie diese z.B. in der Druckschrift WO2014/170184 A1 beschrieben ist, wobei das Kohlenstoffdioxid aus dem Luft-/Gasstrom in der Kohlenstoffdioxidgewinnungsanlage zunächst mittels eines Adsorptionsmaterials, welches vorzugsweise mit Amingruppen (primäre, sekundäre, tertiäre Amine) funktionalisiert (kovalente oder physikalische Bindung der Amine) ist, bei Umgebungstemperatur und -druck adsorbiert wird, wobei zur Umkehrung des Adsorptionsprozesses und damit zur Freigabe des adsorbierten Kohlenstoffdioxides ein Temperatur-Vakuum-Wechselverfahren durchgeführt wird, wobei hierzu Wärme in das Adsorbermaterial eingebracht wird und der Druck um das Adsorptionsmaterial abgesenkt wird und die Wärme wenigstens teilweise aus der exothermen Methan- oder Fischer- Tropsch-Synthese oder aus einer anderen geeigneten Kohlenwasserstoffsynthese entnommen wird. Weiter wird während der Kohlenstoffdioxid Adsorption aus einem feuchten Luft-/Gasstrom Wasser auf dem Adsorptionsmaterial adsorbiert, welches ebenfalls durch das Temperatur- Vakuum-Wechselverfahren regeneriert wird und danach entweder in der Kohlstoffdioxidgewinnungsanlage oder der Elektrolyseanordnung nutzbar gemacht werden

kann oder abgeschieden wird. Auf diese Weise werden zwei eigentlich voneinander getrennt arbeitende Anlagen zu einer Gesamtanlage zusammen geführt, wobei das erfinderische Verfahren die Abfallwärme der Syntheseanlage energetisch genutzt wird, um die notwendige thermische Desorption durchzuführen.

Die Wärme wird aus in dem Syntheseprozess anfallendem Dampf entnommen. Die dort enthaltene Wärme hat eine hohe Dichte und kann auch bedarfsweise zwischengespeichert werden.

Die Elektrolyseanordnung ist eine mit regenerativ erzeugter Elektroenergie arbeitende Wasserdampfelektrolyse (SOEC) zur Erzeugung von Wasserstoff, aus der anfallende Wärme aus der Kühlung des Sauerstoff- und/oder Wasserstoffgasstroms zur Umkehrung des $CO_2$-Adsorptionsprozesses entnommen wird. Die Nutzung der Wärme aus der Wasserdampfelektrolyse alleine reicht bereits aus, um ca. bis zu 35 % des Wärmebedarfs der Kohlenstoffdioxidgewinnungsanlage im erfinderischen Sinne zu decken.

Im Syntheseverfahren anfallendes überschüssiges und/oder nicht verwendbares Restgas wird verbrannt und ein sich bildendes Rauchgas stofflich und/oder energetisch genutzt, wobei hierzu die Wärme des Rauchgases zur Umkehrung des Adsorptionsprozesses genutzt wird und/oder das $CO_2$-haltige Rauchgas als Luft-/Gasstrom oder als Zusatz in dem Luft-/Gasstrom eingesetzt wird.

Insbesondere kann Niedertemperaturabwärme mit Temperaturen unterhalb von 80°C aus den unterschiedlichsten Bereichen der Anlage als auch von externen Quellen zur Regeneration des adsorbierten Wassers in der Kohlenstoffgewinnungsanlage eingesetzt werden, wodurch ein Großteil des adsorbierten Wassers regeneriert und damit der Wärmeverbrauch im Bereich von 90-130°C deutlich reduziert werden kann.

Es wird ein Wärmepumpenprozess zur Erhöhung der Temperatur von Wärme aus dem Syntheseprozess und/oder Restgasnutzung und/oder Kühlung in der Elektrolyse und/oder weiterer Prozessabwärme verwendet. Es wird also vorgeschlagen, Wärme oder Abwärme aus unterschiedlichen Quellen, deren Temperaturniveau für die Desorption des Kohlenstoffdioxides zu niedrig ist, durch einen Wärmepumpenprozess im Temperaturniveau aufzuwerten und damit für die Desorption nutzbar zu machen. Damit wird die Nutzung von auch geringer Abwärme der Teilprozesse allgemein überhaupt erst für den Desorptionsprozess nutzbar, wobei lediglich ein geringer Energieeinsatz für den Wärmepumpenprozess notwendig ist.

Die Kohlenstoffdioxidgewinnungsanlage wird bevorzugt kontinuierlich betrieben, wobei der Kohlenstoffdioxidbedarf des Syntheseprozesses bevorzugt, aufgrund von Schwankungen von zur Verfügung stehender regenerativ erzeugter Elektroenergie für die Wasserstofferzeugung, diskontinuierlich erfolgt und das gewonnene Kohlenstoffdioxid in einem Pufferspeicher zwischengespeichert wird. Unter einem kontinuierlichen Kohlenstoffdioxidgewinnungsanlagenbetrieb ist eine aus mehreren kleineren Kohlenstoffdioxidgewinnungsanlagen bestehende Gesamtanlage zu sehen, deren Gesamtleistung entsprechend ausgebildet ist und deren einzelne notwendige thermische Desorptionen zeitlich nacheinander durchgeführt werden, so dass ein quasi kontinuierlicher Betrieb ausgebildet ist.

Das Zwischenpuffern des Kohlenstoffdioxides in einem Kurzzeitspeicher und einem parallel dazu geschalteten Langzeitspeicher erfolgt mit erhöhtem Druck.

Das Langzeitzwischenpuffern kann über ein Verflüssigen des Kohlenstoffdioxides erfolgen. Der Syntheseprozess erfolgt aufgrund von Schwankungen von zur Verfügung stehender regenerativ erzeugter Elektroenergie diskontinuierlich und die nutzbare Wärme aus der Synthese und der Elektrolyse für die Kohlenstoffdioxidgewinnungsanlage kann in einem Pufferspeicher zwischengespeichert werden.

Bei Betrieb der Kohlenstoffdioxidgewinnungsanlage kann sich innerhalb der Kohlenstoffdioxidanlage angereichertes Wasser gewonnen werden, wobei dieses Wasser aus der Atmosphäre stammt und für die Elektrolyse zur Herstellung von Wasserstoff eingesetzt werden kann. Hierbei entstehen in der Regel mindestens 1 Mol Wasser pro 1 Mol bereitgestelltem Kohlenstoffdioxid. Hierdurch kann ein Großteil des Wasserbedarfs der Synthese bereits durch die Kohlenstoffdioxidgewinnungsanlage gedeckt werden. Dieser Aspekt stellt somit eine weitere eigenständige Optimierung, die zusätzlich gesehen werden kann, dar.

Eine bevorzugte Ausführungsvariante ist gegeben, wenn zusätzlich zu Wasserstoff noch Kohlenmonoxid aus Kohlenstoffdioxid mit Hilfe der mit regenerativ erzeugter Elektroenergie betriebenen Elektrolyseanordnung gewonnen wird.

Ferner kann diesbezüglich die Elektrolyseanordnung eine Wasserdampfelektrolyse sein.

Als weitere Ergänzung zu der Gewinnung von Kohlenmonoxid aus Kohlenstoffdioxid mit Hilfe der mit regenerativ erzeugter Elektroenergie betriebenen Elektrolyseanordnung zusätzlich zu Wasserstoff, kann der Dampf direkt aus dem während des Regenerationsbetriebes der $CO_2$-Gewinnungsanlage aus dem Behälter abgesaugten Kohlenstoffdioxid-Wasserdampf-Gemisch gewonnen werden, ohne dass der Wasserdampf zwischenzeitlich kondensiert wird. Insbesondere ist dies auch in Kombination mit einer Wasserdampfelektrolyse von Vorteil.

Anordnungsgemäß weist die Herstellungsanlage zur Produktion von synthetisch hergestelltem Methan / gasförmigen und/oder flüssigen Kohlenwasserstoffen, insbesondere zur Durchführung des Herstellungsverfahrens eine mit regenerativ erzeugter Elektroenergie betriebene Elektrolyseanordnung zur Herstellung von Wasserstoff, eine Kohlenstoffdioxidgewinnungsanlage zur Herstellung von Kohlenstoffdioxid aus einem Luftstrom, wobei die Kohlenstoffdioxidgewinnungsanlage mit einem Adsorbermaterial arbeitet, das mittels der thermischen Desorption das gebundene Kohlenstoffdioxid wieder abgibt und eine Methan- oder Fischer-Tropsch-Synthese oder andere geeignete Kohlenwasserstoffsynthese zur Synthetisierung von Wasserstoff und Kohlenstoffdioxid zu Methan / gasförmigen und/oder flüssigen Kohlenwasserstoffen auf, wobei Wärmezufuhrmittel und/oder Wärmespeicher zur Zuführung und/oder Zwischenspeicherung von Wärme von der Synthese und/oder Wärme der Elektrolyseanordnung und/oder Wärme einer Verbrennungseinrichtung für Restgase aus dem Syntheseprozess zur Kohlenstoffdioxidgewinnungsanlage vorgesehen sind, wobei die Wärme genutzt wird, um die thermische Desorption durchzuführen.

An dem Methan- oder Fischer-Tropsch-Synthesereaktor oder dem Reaktor einer anderen geeigneten Kohlenwasserstoffsynthese ist zur Abführung der Reaktionswärme eine Dampftrommel zur Trennung des den Synthesereaktor verlassenden dampfhaltigen Siedewassers in Siedewasser und Sattdampf vorgesehen, wobei Zufuhrmittel für den Sattdampf zu einem Wärmeüberträger eines Heizmittelkreislaufes der Kohlenstoffdioxidgewinnungsanlage vorgesehen sind.

Die Elektrolyseanordnung ist eine Wasserdampfelektrolyse (SOEC - Solid Oxide Electrolysis Cell), wobei Zufuhrmittel zum Transport von Wärme aus einer Kühlung des gebildeten Wasserstoff- /Sauerstoffstroms zu einem Wärmeüberträger eines Heizmittelkreislaufes der Kohlenstoffdioxidgewinnungsanlage vorgesehen sind.

Eine Wärmepumpenanordnung ist zur Erhöhung der Temperatur von nutzbarer Abwärme aus der Elektrolyseanordnung, dem Synthesereaktor und/oder Verbrennungseinrichtung für Restgase vorgesehen.

Es sind Speichermittel zur Speicherung und Zuführung zur Methan- oder Fischer-Tropsch- Synthese oder zu einer anderen geeigneten Kohlenwasserstoffsynthese des in der Kohlenstoffdioxidgewinnungsanlage kontinuierlich produzierten Kohlenstoffdioxids vorgesehen. Da der Anfall dieser Überschusselektroenergie aus regenerativ (Wnd, Sonne, Wasser, etc.) erzeugter Elektroenergie tageszeitlich und wetterbedingt schwankt, bedeutet das, dass der Kohlenstoffdioxidbedarf und der Wärmeanfall ebenfalls nicht konstant sind. Die Anlage zur Gewinnung von Kohlenstoffdioxid aus Umgebungsluft wird dagegen mit konstanter Leistung betrieben, so dass zur Anpassung des schwankenden Kohlendioxidbedarfs und Wärmeanfalls Gaspuffer zur Speicherung von Kohlenstoffdioxid und Wärmespeicher in einer besonderen Ausgestaltung notwendig sind. Die Speichermittel weisen eine Kurzzeitgaspufferspeicheranordnung und/oder eine Langzeitgaspufferspeicheranordnung auf.

Nachfolgend werden Ausführungsbeispiele der Erfindung anhand der beiliegenden Zeichnungen in der Figurenbeschreibung detailliert beschrieben, wobei diese die Erfindung erläutern sollen und nicht beschränkend zu werten sind:

Es zeigen:

Fig. 1 eine schematische Darstellung eines Ausführungsbeispiels eines Kohlenstoffdioxid- Gewinnungsanlagenteils;
Fig. 2 eine schematische Darstellung eines Ausführungsbeispiels eines Methanerzeugungsanlagenteils;
Fig. 3 eine schematische Darstellung eines Ausführungsbeispiels eines Syntheseanlagenteils mittels Fischer-Tropsch-Synthese;
Fig. 4 eine schematische Darstellung eines Ausführungsbeispiels einer Wasserdampf-Elektrolyse auf Basis einer SOEC (Solid Oxide Electrolysis Cell),
Fig. 5 eine schematische Darstellung des Regenerationsbetriebes eines Ausführungsbeispiels;
Fig. 6 eine schematische Darstellung eines Ausführungsbeispiels einer Co-Elektrolyse für Fischer-Tropsch-Synthese und
Fig. 7 eine schematische Darstellung eines weiteren Ausführungsbeispiels einer Co-Elektrolyse für Methanerzeugung.

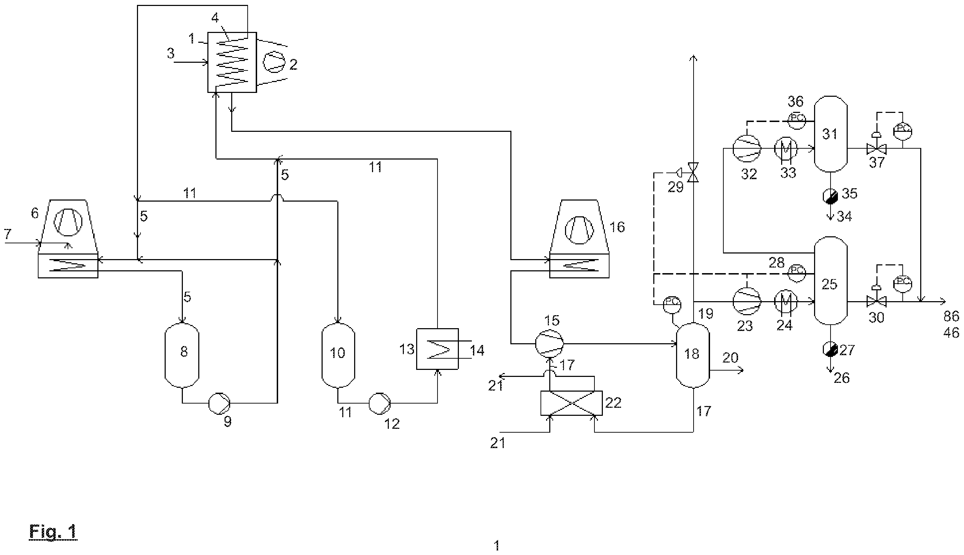

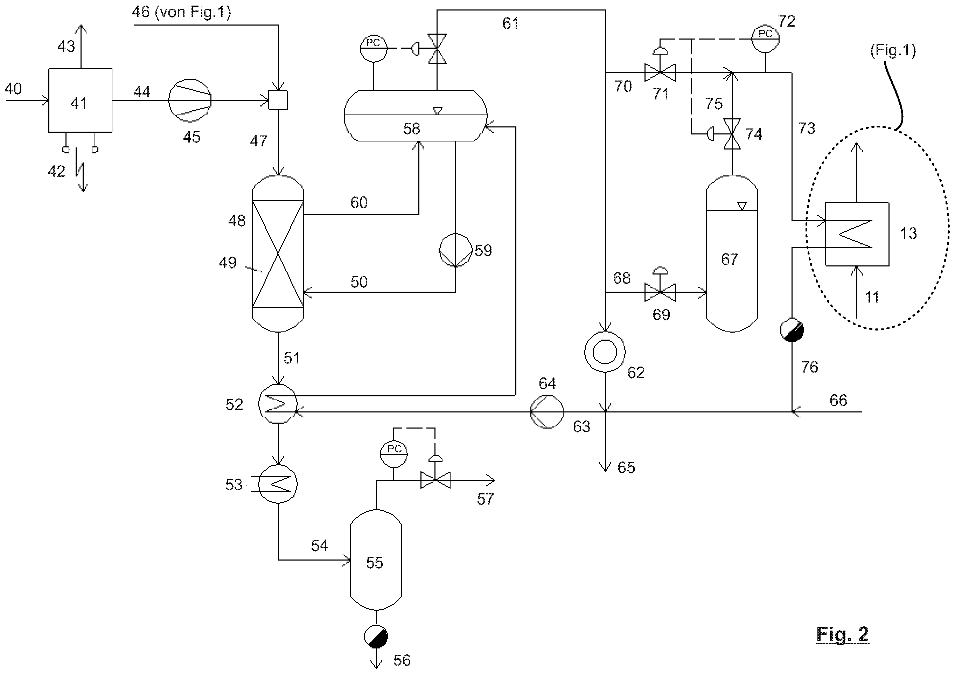

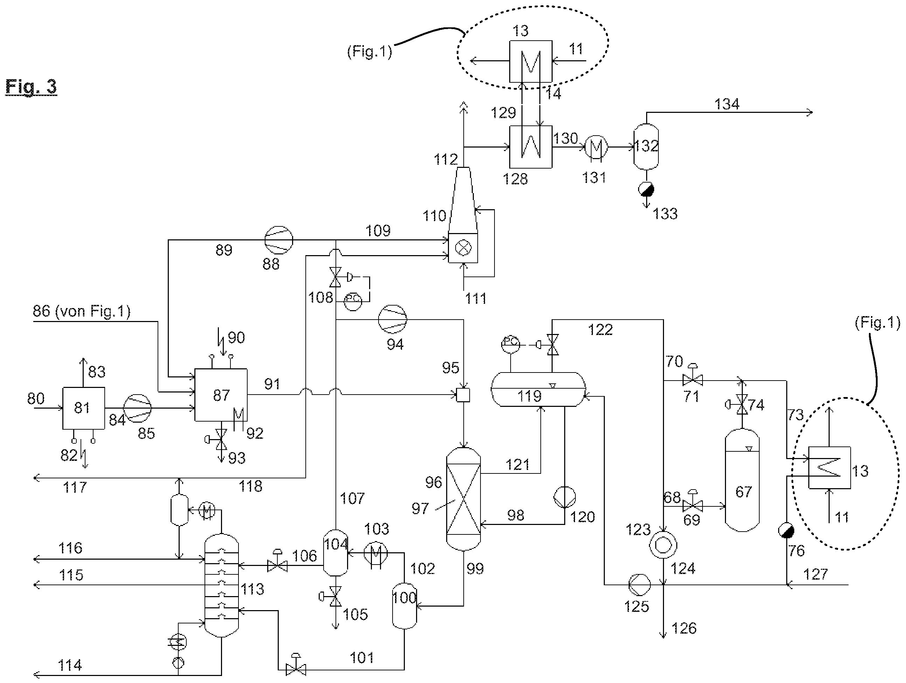

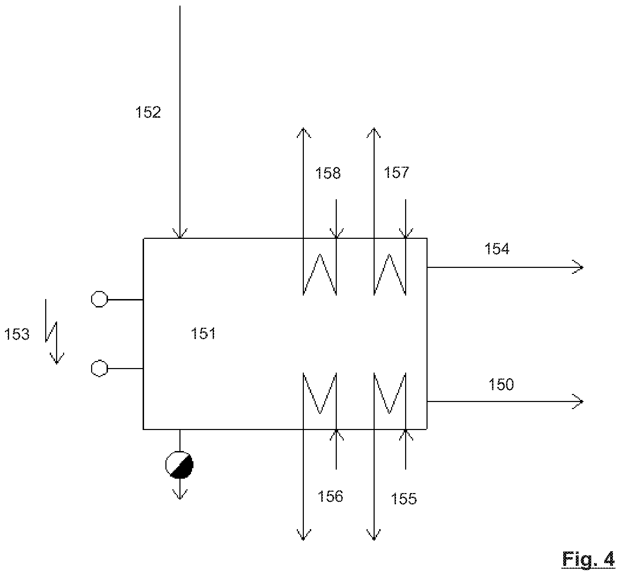

An dieser Stelle soll darauf hingewiesen werden, dass die Gesamtanlage aus dem Kohlenstoffdioxid-Gewinnungsanlagenteil (Fig. 1) und dem Anlagenteil zur Methanisierung (Fig. 2) bzw. zur Synthetisierung (Fig. 3) besteht. Die Wasserdampfelektrolyseeinheit (Fig. 4) kann sowohl in dem Methanisierungsanlagenteil (Fig. 2) als auch in dem Synthetisierungsanlagenteil mit Fischer-Tropsch-Synthese (Fig. 3) eingesetzt werden.

In Fig. 1 ist eine schematische Darstellung eines Ausführungsbeispiels eines Kohlenstoffdioxid- Gewinnungsanlagenteil (Fig. 1) dargestellt.

Der Kohlenstoffdioxid-Gewinnungsanlagenteil (Fig. 1) dient der Gewinnung von Kohlenstoffdioxid 19 aus der Umgebungsluft und stellt bei

Bedarf ebenfalls kondensiertes Wasser 20 aus der Umgebungsluft für weitere stoffliche Nutzung zur Verfügung. Hierbei wurde ein Kohlenstoffdioxid-Gewinnungsanlagenteil gewählt, der Kohlenstoffdioxid zunächst über einen Adsorptionsbetrieb aus dem Luftstrom bindet und im Anschluss durch ein Temperatur-Vakuum-Wechselverfahren das Kohlenstoffdioxid zur weiteren Nutzung freigibt.

Adsorptionsbetrieb:

Durch einen mit Adsorbermaterial gefüllten Behälter 1 wird mittels Gebläse 2 Umgebungsluft 3 gesaugt. Die Umgebungsluft enthält üblicherweise 0,04 Vol.-% Kohlenstoffdioxid und je nach Klima eine gewisse Menge Wasserdampf. Das Kohlenstoffdioxid wird zu einem hohen Anteil an der Oberfläche des Adsorbermaterials, welches vorzugsweise mit Amingruppen funktionalisiert ist, angereichert. Weiter reichert sich Wasser an der Oberfläche des Adsorptionsmaterials an, wobei gewöhnlich mindestens 2 Mol Wasser pro 1 Mol Kohlenstoffdioxid, mindestens jedoch 1 Mol pro 1 Mol Kohlenstoffdioxid, adsorbiert wird.

Regenerationsbetrieb mittels Temperatur-Vakuum-Wechselverfahren:
Ist die Oberfläche des Adsorbermaterials 1 mit Kohlenstoffdioxid gesättigt bzw. angereichert, muss es regeneriert werden.

Zunächst wird Behälter 1 auf einen Druck von 20-400 $mbar_{abs}$ evakuiert und anschliessend wird aus dem Heizmittelbehälter 10 Heizflüssigkeit 11 mit einer Temperatur von 20-120°C mittels Heizmittelpumpe 12 durch einen Wärmeübertrager 13 gepumpt.

Im Wärmeübertrager 13 wird die Heizflüssigkeit 11 durch eine externe Wärmequelle 14 (die Wärme stammt erfindungsgemäß aus den Teilanlagen gem. Fig. 2 oder Fig. 3 oder Fig. 4) auf 80-130°C aufgeheizt. Anschließend gelangt die Heizflüssigkeit in den Behälter 1, wo sie über die Wärmeübertragerflächen 4 die Wärme an das Adsoptionsmaterial abgibt. Durch die Wärmezufuhr wird das gebundene Kohlenstoffdioxid und Wasser desorbiert und aus dem Behälter 1 mittels Flüssigkeitsringverdichter 15 abgesaugt.

Die abgekühlte Heizflüssigkeit 11 läuft zurück in den Heizmittelbehälter 10, aus dem sie erneut dem Prozess zugeführt werden kann.

Das heiße abgesaugte Gemisch aus Kohlenstoffdioxid und Wasserdampf wird in einem Kühler (z.B. Kühlturm 16) gekühlt und gelangt über den Flüssigkeitsringverdichter 15, in das kaltes Kondensat 17 zur Verbesserung der Saugwirkung und zur weiteren Abkühlung gegeben wird, in den Trennbehälter 18.

Im Trennbehälter 18 wird das Kohlenstoffdioxid 19 vom Kondensat 17/20, das aus der Luftfeuchtigkeit stammt und sich ebenfalls am Adsorbermaterial abgesetzt hatte, durch Schwerkraftwirkung getrennt. Ein Teil des Kondensates wird nach Kühlung mit Kühlwasser 21 im Kühler 22 dem Flüssigkeitsringverdichter zugeführt. Das Überschusskondensat 20 wird nach dem Trennbehälter 18 abgegeben.

Das hochkonzentrierte Kohlenstoffdioxid 19 wird der weiteren Nutzung in den Anlagenteilen gemäß Fig. 2 oder Fig. 3 zugeführt.

Nach dem Regenerationsprozess werden die Wärmeübertragerflächen 4 von einer Kühlflüssigkeit durchströmt. Die Kühlflüssigkeit 5 nimmt die sensible Wärme des Adsorptionsmaterials und des Behälter 1 auf und führt diese aus Behälter 1 ab. Die aufgeheizte Kühlflüssigkeit gelangt anschließend in einen Kühlturm 6, wo die aufgenommene Wärme wieder an die Umgebung abgegeben wird. Zur Verbesserung der Kühlwirkung, insbesondere bei hohen Umgebungstemperaturen, kann im Kühlturm auf die Wärmeübertragerflächen des Kühlturms Wasser 7 verdüst werden, das dort verdunstet.

Die kalte Kühlflüssigkeit 5 wird in einem Kühlmittelbehälter 8 gesammelt und von dort mit Hilfe einer Kühlmittelpumpe 9 wieder dem Adsorberbehälter 1 zugeführt.

Nach der Kühlung von Behälter 1 und des Adsorptionsmaterials unter eine Temperatur von maximal 50°C wird Behälter 1 belüftet und es kann erneut mit dem Adsorptionsprozess begonnen werden.

Alternativer Regenerationsbeschrieb mittels Temperatur-Vakuum-Wechselverfahren zur Einkopplung von Abwärme sowohl im Temperaturbereich 80-130°C als auch im Temperaturbereich 50-90°C:
Ist die Oberfläche des Adsorbermaterials 1 mit Kohlenstoffdioxid gesättigt bzw. angereicht, muss es regeneriert werden.

Zunächst wird Behälter 1 auf einen Druck von 20-400 $mbar_{abs}$ evakuiert und anschliessend aus dem Heizmittelbehälter 10 Heizflüssigkeit 11 mit einer Temperatur von 20-50°C mittels Heizmittelpumpe 12 durch einen Wärmeübertrager, der seriell zu dem Wärmeüberträger 13/14 ergänzend angeordnet ist, gepumpt.

Im seriell ergänzend geschalteten Wärmeübertrager wird die Heizflüssigkeit 11 durch eine externe Wärmequelle, wobei die Wärme erfindungsgemäß aus den Teilanlagen gem. Fig. 2 oder Fig. 3 oder Fig. 4 stammt und auf 45-85°C aufgeheizt ist. Anschließend gelangt die Heizflüssigkeit in den Behälter 1, wo sie über die Wärmeübertragerflächen 4 die Wärme an das Adsoptionsmaterial abgibt. Durch die Wärmezufuhr werden Teile des adsorbierten Wassers und Teile des adsorbierten $CO_2$ desorbiert (wobei typischerweise im angegebenen Temperaturbereich die Wasserdesorption bevorzugt vor der $CO_2$-Desorption stattfindet) und aus dem Behälter 1 mittels Flüssigkeitsringverdichter 15 abgesaugt.

Die abgekühlte Heizflüssigkeit 11 läuft zurück in den Heizmittelbehälter 10, aus dem sie erneut dem Prozess zugeführt werden kann.

Anschliessend wird aus dem Heizmittelbehälter 10 Heizflüssigkeit 11 mit einer Temperatur von 70-120°C mittels Heizmittelpumpe 12 durch einen Wärmeübertrager 13 gepumpt.

Im Wärmeübertrager 13 wird die Heizflüssigkeit 11 durch eine externe Wärmequelle 14 (die Wärme stammt erfindungsgemäß aus den Teilanlagen gem. Fig. 2 oder Fig. 3 oder Fig. 4) auf 80-130°C aufgeheizt. Anschließend gelangt die Heizflüssigkeit in den Behälter 1, wo sie über die Wärmeübertragerflächen 4 die Wärme an das Adsoptionsmaterial abgibt. Durch die Wärmezufuhr wird vor allem Kohlenstoffdioxid desorbiert und aus dem Behälter 1 mittels Flüssigkeitsringverdichter 15 abgesaugt.

Die abgekühlte Heizflüssigkeit 11 läuft zurück in den Heizmittelbehälter 10, aus dem sie erneut dem Prozess zugeführt werden kann.

Das heiße abgesaugte Kohlenstoffdioxid wird im Kühlturm 16 gekühlt und gelangt über den Flüssigkeitsringverdichter 15, in das kalte Kondensat 17 zur Verbesserung der Saugwirkung und zur weiteren Abkühlung gegeben wird, in den Trennbehälter 18.

Im Trennbehälter 18 wird das Kohlenstoffdioxid 19 vom Kondensat 17/20, das aus der Luftfeuchtigkeit stammt und sich ebenfalls am Adsorbermaterial abgesetzt hatte, durch Schwerkraftwirkung getrennt. Ein Teil des Kondensates wird nach Kühlung mit Kühlwasser 21 im Kühler 22 dem Flüssigkeitsringverdichter zugeführt. Das Überschusskondensat 20 wird nach dem Trennbehälter 18 abgegeben.

Das hochkonzentrierte Kohlenstoffdioxid 19 wird der weiteren Nutzung in den Anlagenteilen gemäß Fig. 2 oder Fig. 3 zugeführt.

Nach dem Regenerationsprozess werden die Wärmeübertragerflächen 4 von einer Kühlflüssigkeit durchströmt. Die Kühlflüssigkeit 5 nimmt die sensible Wärme des Adsorptionsmaterials und des Behälter 1 auf und führt diese aus Behälter 1 ab. Die aufgeheizte Kühlflüssigkeit gelangt anschließend in einen Kühlturm 6, wo die aufgenommene Wärme wieder an die Umgebung abgegeben wird. Zur Verbesserung der Kühlwirkung, insbesondere bei hohen Umgebungstemperaturen, kann im Kühlturm auf die Wärmeübertragerflächen des Kühlturms Wasser 7 verdüst werden, das dort verdunstet.

Die kalte Kühlflüssigkeit 5 wird in einem Kühlmittelbehälter 8 gesammelt und von dort mit Hilfe einer Kühlmittelpumpe 9 wieder dem Adsorberbehälter 1 zugeführt.

Nach der Kühlung von Behälter 1 und des Adsorptionsmaterials unter eine Temperatur von maximal 50°C wird Behälter 1 belüftet und es kann erneut mit dem Adsorptionsprozess begonnen werden.

Kohlenstoffdioxidbereitstellung für die folgende Nutzung in einer Methan- oder Fischer-Tropsch-Synthese oder anderen geeigneten Kohlenwasserstoffsynthese:

Durch die erforderliche Regeneration des Adsorptionsmaterials kann mit einem Prozesskreis keine kontinuierliche Kohlenstoffdioxidabtrennung aus der Luft erfolgen. Eine Parallelschaltung mehrerer Adsorptionsbehälter 1 und die dazugehörenden Kühl- und Heizkreise und ein zeitlich versetzter Betrieb ermöglichen jedoch sehr wohl die Überführung des diskontinuierlichen in einen quasikontinuierlichen Prozess, so dass diese Ausgestaltung eine besonders bevorzugte Ausgestaltung darstellt.

Das aus der $CO_2$-Gewinnung quasikontinuierlich bei Umgebungsdruck anfallende Kohlenstoffdioxid 19 wird mit einem Verdichter 23 und einer anschließenden Gaskühlung 24 zur Abführung der Verdichterwärme auf einen Druck verdichtet, der höher ist, als der Druck in den nachfolgenden Syntheseanlagen, und anschließend in einem Gaspuffer 25 gespeichert.

Das aufgrund der Kühlung anfallende Wasserkondensat 26 wird mit einem Kondensatableiter 27 aus dem Gaspuffer 25 abgeführt.

Die Regelarmatur 29 dient zur Regelung des Druckes im Behälter 18, der von der zum Verdichter 23 abgeführten Menge $CO_2$ abhängt.

Entnimmt der Verdichter 23 weniger $CO_2$ aus dem Behälter 18 als in der $CO_2$-Gewinnung produziert wird, öffnet das Regelventil 29 und führt die überschüssige $CO_2$-Menge an die Atmosphäre oder einen anderen Zwischenspeicher, z.B. einen Gassack, ab.

Entnimmt der Verdichter 23 mehr $CO_2$ als produziert wird, schließt zunächst die Regelarmatur 29, anschließend wird zusätzlich die Förderleistung des Verdichters 23 reduziert, so dass der Druck im Behälter 18 konstant bei einem vorgegebenen Wert bleibt.

Hat der Behälterdruck 28 seinen maximal zulässigen Wert erreicht, wird der Verdichter 23 abgeschaltet.

Die Entnahme des Kohlenstoffdioxids aus dem Behälter 25 erfolgt über das Regelventil 30, das den Druck in der nachfolgenden Syntheseanlage konstant bei einem vorgegebenen Wert hält.

Sinkt der Druck im Behälter 25 unter einen zulässigen Wert, der höher als der Druck in der nachfolgenden Syntheseanlage sein muss, schaltet der Verdichter 23 wieder zu und füllt den Gaspuffer 25.

Der Unterschied zwischen Pufferdruck und Entnahmedruck, das Puffervolumen und die pro Zeiteinheit benötigte $CO_2$-Menge bestimmen die mit dem Puffer überbrückbare Zeit.

Da die zusätzliche Druckerhöhung für die Gaspufferung über den notwendigen Betriebsdruck der Syntheseanlage hinaus zusätzliche Verdichtungsenergie kostet, sollte aus Wirtschaftlichkeitsgründen diese nicht zu hoch gewählt werden.

Das bedeutet, dass längere Überbrückungszeiten vor allem durch das Puffervolumen bestimmt werden. Da dieses begrenzt ist, eignet sich ein solcher Speicher mit geringer Drucküberhöhung nur als Kurzzeitspeicher um kurzzeitige Regelschwankungen auszugleichen.

Um einen höheren $CO_2$-Bedarf für längere Zeiten abzusichern, wird vorgeschlagen, parallel zu dem Kurzzeitspeicher 25 einen Langzeitspeicher 31 zu errichten, der bei einem höheren Druck arbeitet.

Dieser Langzeitspeicher 31 wird mit dem Verdichter 32 und dem anschließenden Kühler 33 zur Kondensation der Feuchtigkeit im Gas, die als Kondensat 34 über den Kondensatableiter 35 aus dem Behälter 31 abgeführt wird, mit $CO_2$ aus dem Behälter 25 auf einen Druck weit oberhalb des Betriebsdruckes der Syntheseanlage und des maximalen Druckes des Behälters 25 gefüllt.

Der Verdichter 32 ist nur in Betrieb, wenn der Behälter 25 seinen maximalen Druck bereits erreicht hat. In diesem Fall wird die Leistung des Verdichters 23 nicht reduziert, sondern zusammen mit dem Verdichter 32 mit Nennleistung weiter betrieben. Hat der Behälter 31 den maximalen Druck 36 erreicht, wird der Verdichter 32 abgeschaltet und die Leistung des Verdichters 23 entsprechend dem Verbrauch in der Syntheseanlage reduziert.

In Zeiten eines erhöhten $CO_2$-Bedarfs in der Syntheseanlage wird zunächst das Kohlenstoffdioxid aus dem Behälter 25 über das Regelventil 30 entnommen. Ist der Druck im Behälter unter den Betriebsdruck der Syntheseanlage abgesunken, d.h. ist der Behälter 25 leer, öffnet das Regelventil 37 und versorgt die Syntheseanlage mit $CO_2$ aus dem Langzeitspeicher 31.

Bei Reduzierung des $CO_2$-Bedarfs in der Syntheseanlage schließt zuerst das Regelventil 37, dann steigt der Druck 28 im Behälter 25 bis auf den maximal zulässigen Wert. Anschließend startet der Verdichter 32 und füllt den Puffer 31 bis zum maximalen Druckwert 36, bei dem der Verdichter 32 abschaltet. Bei weiterem Überangebot an $CO_2$ wird schließlich die Förderleistung des Verdichters 23 verringert.

Eine weitere Steigerung der Puffermenge $CO_2$ ist möglich, wenn das $CO_2$ verflüssigt und flüssig gelagert wird. Aufgrund der höheren Dichte ist damit bei gleichem Volumen eine höhere Menge $CO_2$ speicherbar.

Die Flüssigspeicherung stellt einen Sonderfall des Langzeitspeichers dar und wird nicht extra ausführlich behandelt, gleichwohl ist diese Ausgestaltung von großer Bedeutung.

**Fig. 2** zeigt eine schematische Darstellung eines Ausführungsbeispiels eines Methanerzeugungsanlagenteils.

Die Produktion von Methan aus Kohlenstoffdioxid und Wasserstoff, hergestellt in einer Elektrolyse mit Hilfe von regenerativ erzeugter Elektroenergie, erfolgt nach der Reaktion von Sabatier:

$$CO_2 + 4\,H_2 \rightarrow CH_4 + 2\,H_2O \quad \Delta H_R = -165\ kJ/mol \qquad\qquad R1$$

Deionisiertes Wasser 40 wird in einem Elektrolyseapparat 41 mit regenerativ erzeugter Elektroenergie 42 in Sauerstoff 43 und Wasserstoff 44 zerlegt. Der Sauerstoff 43 wird einer Nutzung zu- oder ungenutzt an die Umgebung abgeführt.

Der Wasserstoff 44 wird in einem Verdichter 45 auf Prozessdruck verdichtet und mit Kohlenstoffdioxid 46, das bereits bei Prozessdruck zur Verfügung steht, vermischt und als Mischgas 47 einem Synthesereaktor 48 zugeführt.

Der Synthesereaktor 48 ist mit einem geeigneten Katalysator 49 in ausreichender Menge gefüllt und, da die Sabatierreaktion exotherm ist, mit einem Siedewasserkreislauf 50 gekühlt.

Die Kühltemperatur und damit auch der Siedewasserdruck sind so gewählt, dass ein möglichst hoher Umsatz des Kohlenstoffdioxids zu Methan erfolgt.

Dieser hohe Umsatz wird in der Regel erreicht, wenn das Reaktionsgas 51 mit einer Temperatur von ca. 250 bis 300°C den Reaktor 48 verlässt.

Nach dem Reaktor 48 wird das Reaktionsgas möglichst auf niedrige Temperaturen abgekühlt, um das Reaktionswasser aus dem Gasgemisch abzutrennen. Die Abkühlung erfolgt zunächst in einem Wärmeübertrager 52, in dem kaltes Speisewasser vorgewärmt wird. Die erforderlichen niedrigen Temperaturen werden erst in einem mit Kühlwasser betriebenen Kühler 53 erreicht.

Das kalte Gasgemisch 54 gelangt in einen Abscheider 55, in dem das Reaktionswasser 56 aus dem methanreichen Gas 57 abgetrennt wird.

Das erzeugte Methan 57 wird der gewünschten weiteren Nutzung zugeführt.

Das zur Kühlung eingesetzte Siedewasser 50 wird der Dampftrommel 58 entnommen und mittels Pumpe 59 dem Reaktor 48 zur Abführung der Reaktionswärme zugeführt. Die Umlaufmenge an Siedewasser 50 wurde so gewählt, dass nur ein geringer Teil des Siedewassers infolge der Wärmeaufnahme verdampft. Das den Reaktor verlassende dampfhaltige Siedewasser gelangt in die Dampftrommel 58, in der die Trennung zwischen Siedewasser und Sattdampf erfolgt. Der Sattdampf 61 wird druckgeregelt aus der Dampftrommel 58 abgeführt und einer Nutzung z.B. einer Wärmesenke 62 zugeführt. Das aus der Wärmenutzung zurückgegebene Dampfkondensat wird mittels Pumpe 64 komprimiert, im Wärmeübertrager 52 vorgewärmt und wieder in die Dampftrommel 58 gegeben. Eventuell auftretender Dampfkondensatverlust 65 kann durch neues Speisewasser 66 ersetzt werden.

Erfindungsgemäß wird das in der $CO_2$-Gewinnungsanalge (Fig. 1) aus der Luft 3 abgetrennte Kohlenstoffdioxid 19 als Kohlenstoffdioxid 46 für die Erzeugung von Methan 57 (Fig. 2) oder als Kohlenstoffdioxid 86 für die Erzeugung von flüssigen Kohlenwasserstoffen (114, 115, 116, 117) (Fig. 3) mit Hilfe von Wasserstoff 44 bzw. 84, hergestellt in einer Wasserelektrolyse 41 bzw. 81 mit Hilfe von regenerativ erzeugter Elektroenergie (42, 82), eingesetzt. Weiterhin wird der im Syntheseprozess (Fig. 2 bzw. Fig. 3) anfallende Dampf 61 bzw. 122 als Wärmequelle 14 für die Desorption des an den Adsorbermaterial angelagerten Kohlenstoffdioxids verwendet.

Wärmespeicher zur Speicherung von Wärme aus dem Syntheseprozess (Vgl. Fig. 2 und Fig. 3): Die Beschreibung erfolgt am Beispiel der Methansynthese gemäß Fig. 2. Die Dampfspeicherung in der Synthese von flüssigen Kohlenwasserstoffen gemäß Fig. 3 ist analog der Dampfspeicherung in der Methansynthese.

Der in der Syntheseanlage diskontinuierlich anfallende Druckdampf 61 wird als Wärmequelle 14 für die Desorption des an den Adsorbermaterialien angelagerten Kohlenstoffdioxids eingesetzt. Durch den diskontinuierlichen Betrieb der Syntheseanlage (Fig. 2/3) auf Grund der unterschiedlichen Verfügbarkeit der regenerativ erzeugten Elektroenergie fällt zeitweise Überschussdampf an, der in der Desorption (Fig. 1) nicht genutzt werden kann. Zu anderen Zeiten, in der die Syntheseanlage (Fig. 2/3) mit geringerer Last betrieben wird, steht für die Wärmeversorgung der Desorption (Fig. 1) zu wenig Dampf zur Verfügung.

Zur Vergleichmäßigung der Wärmeversorgung in der Desorption wird deshalb der in der Syntheseanlage (Fig. 2/3) diskontinuierlich anfallende Druckdampf 61 in einem Wärmespeicher 67 gespeichert. Der Wärmespeicher 67 ist z.B. ein mit Siedewasser gefüllter Gleitdruckspeicher (Ruthsspeicher). Andere geeignete Wärmespeicher sind z.B. Schichtenspeicher, Flüssigsalzspeicher und thermochemische Speicher.

Beim Ladevorgang wird der in der Synthese erzeugte Überschussdruckdampf 68 über die Armatur 69 in den Speicher 67 gegeben. Der restliche Dampf 70 wird über das Drosselventil 71 auf den Druck 72 reduziert und als Heizdampf 73 für die Beheizung der Heizflüssigkeit 11 im Wärmeübertrager 13 eingesetzt. Zu Beginn des Ladevorganges befinden sich im Speicher 67 Siedewasser und Sattdampf bei Dampfabgabedruck 72. Durch die Zuführung von Druckdampf 68 wird das Siedewasser im Behälter 67 aufgeheizt und der Druck im Behälter steigt an. Der maximal mögliche Druck entspricht dem Druck des zugeführten Druckdampfes 68. Durch den Druckanstieg vermindert sich der Dampfanteil und erhöht sich der Wasseranteil im Behälter. Die zugeführte Wärme ist in Form von Siedewasser gespeichert.

Besteht in der $CO_2$-Desorption Wärmemangel, weil die Syntheseanlage (Fig. 2/3) direkt zu wenig Dampf liefert, wird das Drosselventil 74 geöffnet und die gewünschte Differenzdampfmenge 75 aus dem Wärmespeicher 67 entnommen. Durch die Dampfentnahme reduziert sich der Druck im Behälter 67, und es wird Siedewasser im Behälter verdampft. Eine Dampfentnahme ist bis auf den Druck 72 möglich. Nach der Dampfentnahme kann der Wärmespeicher wieder aufgeladen werden.

Das im Wärmeübertrager 13 anfallende Dampfkondensat 76 wird als Speisewasser 63 wieder in den Syntheseprozess zurückgeführt.

Anlagen zur Erzeugung von Methan (Fig. 2) und flüssigen Kohlenwasserstoffen (Fig. 3) aus Kohlenstoffdioxid und Wasserstoff, hergestellt mit regenerativ erzeugter Elektroenergie, dienen in der Regel als Stromspeicher und werden mit regenerativ erzeugter Elektroenergie betrieben, die im Stromnetz überschüssig ist, d.h. die bei Nichtverwendung zur unwirtschaftlichen Abregelung oder sogar zur Abschaltung von konventionellen Stromerzeugern führen würde.

In **Fig. 3** ist eine schematische Darstellung eines Ausführungsbeispiels eines Syntheseanlagenteils mittels Fischer-Tropsch-Synthese gezeigt.

Zur Herstellung von flüssigen Kohlenwasserstoffen aus Kohlenstoffdioxid und Wasserstoff, hergestellt in einer Elektrolyse mit Hilfe von regenerativ erzeugter Elektroenergie, wird eine Fischer-Tropsch-Synthese (Fig. 3) eingesetzt.

Die Umwandlung von Kohlenstoffdioxid und Wasserstoff zu Kohlenwasserstoffen erfolgt in zwei Schritten:

Schritt 1: Reverse Wassergas-Shift-Reaktion zur Herstellung von kohlenstoffmonoxidhaltigem Synthesegas

$$CO_2 + H_2 \rightarrow CO + H_2O \qquad \Delta H_R = +41,2\ kJ/mol \qquad\qquad R2$$

Schritt 2: z.B. Fischer-Tropsch-Synthese zur Herstellung von flüssigen Kohlenwasserstoffen

$$CO + 2\ H_2 \rightarrow -CH_2- + H_2O \qquad \Delta H_R = -156,5\ kJ/mol \qquad\qquad R3$$

Zur Erhöhung der Produktausbeute kann ein Teil der leichten gasförmigen Kohlenwasserstoffe aus der Fischer-Tropsch-Synthese über einen Reformprozess wieder zu Synthesegas aufgearbeitet und dem Fischer-Tropsch-Prozess erneut zugeführt werden:

$$-CH_2- + H_2O \rightarrow CO + 2\ H_2 \qquad \Delta H_R = +156,5\ kJ/mol \qquad\qquad R4$$

Die Reaktionen R2 und R4 laufen parallel in einem Reaktor ab.

Deionisiertes Wasser 80 wird in einem Elektrolyseapparat 81 mit regenerativ erzeugter Elektroenergie 82 in Sauerstoff 83 und Wasserstoff 84 zerlegt. Der Sauerstoff 83 wird einer Nutzung zu- oder ungenutzt an die Umgebung abgeführt.

Der Wasserstoff 84 wird in einem Verdichter 85 auf Prozessdruck verdichtet und zusammen mit Kohlenstoffdioxid 86, das bereits bei Prozessdruck zur Verfügung steht, sowie der mittels Verdichter 88 aus der Fischer-Tropsch-Synthese zurückgeführten kohlenwasserstoffhaltigen Restgase 89 einem Prozess 87 zur Durchführung der reversen Wassergas-Shift-Reaktion (R2) und der Reformierung (R4) zugeführt.

Die für beide endothermen Reaktionen benötigte Prozesswärme wird mittels regenerativ erzeugter Elektroenergie 90 bereitgestellt.

Den Prozess 87 verlässt das Synthesegas 91 mit dem für die Fischer-Tropsch-Synthese erforderlichen Wasserstoff-Kohlenstoffmonoxid-Molverhältnis. Das durch Kühlung 92 abgetrennte überschüssige Reaktionswasser wird als Kondensat 93 aus dem Prozess abgegeben.

Das Synthesegas 91 wird mit im Verdichter 94 rückverdichtetem Kreislaufgas 95 vermischt und dem Fischer-Tropsch-Reaktor 96 zugeführt.

Der Fischer-Tropsch-Reaktor 96 ist mit einem geeigneten Katalysator 97 in ausreichender Menge gefüllt und, da die Synthesereaktion R3 exotherm ist, mit einem Siedewasserkreislauf 98 gekühlt.

Die Kühltemperatur und damit auch der Siedewasserdruck sind so gewählt, dass in Abhängigkeit vom gewählten Katalysator der Umsatz von Kohlenstoffmonoxid und Wasserstoff zu dem gewünschten Produkt erfolgt.

Dieses gewünschte Produkt wird beim Einsatz von Cobaltkatalysatoren in der Regel erreicht, wenn das Reaktionsprodukt 99 mit einer Temperatur von ca. 220°C den Reaktor 96 verlässt. Das Reaktionsprodukt 99 ist ein Gemisch aus Reaktionsgas und langkettigen, flüssigen Kohlenwasserstoffen mit hoher C-Zahl. Dieses Gemisch wird im Hochtemperaturabscheider 100 in flüssige Kohlenwasserstoffe 101 und kohlenwasserstoffhaltiges Gas 102 getrennt.

Das Gas wird im Kühler 103 weiter abgekühlt und im Trennbehälter 104 durch Schwerkraftwirkung in Reaktionswasser 105, flüssige Kohlenwasserstoffe 106 und Restgas 107 getrennt. Die Abkühlung und Trennung kann auch in mehreren Kühl-Trennstufen erfolgen.

Ein Teil 95 des Restgases 107 wird zur Erhöhung des Kohlenstoffmonoxid-Umsatzes in der Fischer-Tropsch-Synthese mit dem Verdichter 94 rückverdichtet und mit dem Synthesegas 91 dem Reaktor 96 erneut zugeführt.

Zur Vermeidung einer zu hohen Anreicherung von nicht an den Fischer-Tropsch-Reaktionen teilnehmenden Gasbestandteilen ($CO_2$, $CH_4$, $C_2$..., $N_2$) muss ein Teilstrom Gas 108 aus dem Fischer-Tropsch-Prozess abgeführt werden.

Dieser Teilstrom 108 kann entweder als Gas 109 in einer Verbrennungseinrichtung 110 mit Luft 111 zu Rauchgas 112 verbrannt werden, das an die Umgebung abgegeben wird, oder teilweise mit dem Verdichter 88 rückverdichtet und als Strom 89 dem Prozess 87 zur Wiederaufarbeitung der Kohlenwasserstoffe zu Synthesegas zugeführt werden. Auch in diesem Fall wird zur Vermeidung von Stickstoffanreicherung im Gesamtprozess ein Teilstrom in der Verbrennungseinrichtung 110 verbrannt.

Die im Fischer-Tropsch-Prozess abgetrennten flüssigen Kohlenwasserstoffe 101 und 106 gelangen in eine ein- oder mehrstufige Produktaufbereitung 113, in der die Kohlenwasserstoffe zu Endprodukten mit unterschiedlichen Eigenschaften aufgearbeitet werden. Endprodukte sind z.B. Wachs 114, Diesel 115, Naphtha 116 und leichte Kohlenwasserstoffgase 117.

Die in der Produktaufbereitung 113 anfallenden Restgase 118 werden in der Verbrennungseinrichtung 110 zusammen mit dem Restgas 109 mit Luft 111 zu Rauchgas 112, das üblicherweise an die Umgebung abgegeben wird, verbrannt. Diesseits wird jedoch das Rauchgas 112 aus der Verbrennung der Restgase in der Anlage zur Herstellung von flüssigen Kohlenwasserstoffen (Fig. 3) als Wärmequelle 14 für die Desorption und als Luftersatz 3 mit höherem Kohlenstoffdioxidgehalt bzw. zur Zumischung zur Luft zur Erhöhung von deren $CO_2$ Gehalt in der $CO_2$-Gewinnung (Fig. 1) verwendet.

Das zur Kühlung des Reaktors 96 eingesetzte Siedewasser 98 wird der Dampftrommel 119 entnommen und mittels Pumpe 120 dem Reaktor 96 zur Abführung der Reaktionswärme zugeführt. Die Umlaufmenge an Siedewasser 98 wurde so gewählt, dass nur ein geringer Teil des Siedewassers infolge der Wärmeaufnahme verdampft.

Das den Reaktor verlassende dampfhaltige Siedewasser 121 gelangt in die Dampftrommel 119, in der die Trennung zwischen Siedewasser und Sattdampf erfolgt.

Der Sattdampf 122 wird druckgeregelt aus der Dampftrommel 119 abgeführt und einer Nutzung z.B. einer Wärmesenke 123 zugeführt.

Das aus der Wärmenutzung zurückgegebene Dampfkondensat 124 wird mittels Pumpe 125 im Druck erhöht und der Dampftrommel 119 wieder zugeführt.

Eventuell auftretender Dampfkondensatverlust 126 kann durch neues Speisewasser 127 ersetzt werden.

Ferner ist aus der Fig. 3 die Wärmenutzung und $CO_2$-Nutzung aus dem Fackelabgas einer Fischer-Tropsch-Syntheseanlage zu entnehmen.

Das aus der Verbrennung der Restgase 109 aus der Fischer-Tropsch-Synthese und der leichten Kohlenwasserstoffe 118 aus der Produktaufbereitung mit Luft 111 stammende Rauchgas 112 wird stofflich und/ oder energetisch in der $CO_2$-Gewinnung (Fig. 1) genutzt, da erkannt wurde, dass dieses Rauchgas 112 als $CO_2$-Lieferant genutzt werden kann. Ferner wurde erkannt, dass auch die thermische Energie der Rauchgase 112 für den Desorptionsprozess genutzt werden kann. Dazu wird das Rauchgas 112 über einen Wärmeübertrager 128 gegeben, der die Wärme 129 über einen Wärmeträgerkreislauf als Wärme 14 dem $CO_2$-Gewinnungsprozess zur Verfügung stellt. Das abgekühlte Rauchgas 130 wird im Kühler 131 weiter abgekühlt und dem Trennbehälter 132 zugeführt, indem das auskondensierte Verbrennungswasser als Abwasser 133 vom Gasstrom 134 abgeschieden wird. Der Gasstrom 134 enthält neben Stickstoff und Sauerstoff aus der Verbrennungsluft 111 noch Kohlenstoffdioxid. Da die $CO_2$-Konzentration im Gas 134 wesentlich höher als in der Umgebungsluft ist, kann dieses der Luftzufuhr in der $CO_2$ Gewinnungsanlage beigemischt werden und damit die $CO_2$-Abtrennung aus diesem Gemisch in der $CO_2$-Gewinnungsanlage schneller und mit geringerem Energieaufwand erfolgen.

**Fig. 4** zeigt eine schematische Darstellung eines Ausführungsbeispiels einer Wasserdampf-Elektrolyse auf Basis einer SOEC (Solid Oxide Electrolysis Cell).

Zur Herstellung von Wasserstoff 150 in einer Wasserdampf-Elektrolyse 151 auf Basis einer SOEC (Solid Oxide Electrolysis Cell) wird Wasserdampf 152 rekuperativ gegen die heißen Produktgase Sauerstoff und Wasserstoff vorgewärmt und bei hohen Temperaturen mit Hilfe von regenerativ erzeugter Elektroenergie 153 in Sauerstoff 154 und Wasserstoff 150 zerlegt.

Die nicht rekuperativ nutzbare Wärme wird mit Kühlwasser 155 und 156 aus dem Wasserstoffstrom sowie 157 und 158 aus dem Sauerstoffstrom abgeführt.

Wird also in der Synthese (Fig. 2 oder Fig. 3) anstelle einer Wasserelektrolyse (41, 81) eine Wasserdampfelektrolyse (Fig. 4) eingesetzt, soll ein Teil der überschüssigen Wärme 155, 156 und 157 und 158 aus der Produktgaskühlung der Wasserdampfelektrolyse 151 als Wärmequelle 14 für die Desorption des Kohlenstoffdioxids eingesetzt werden.

Ferner sind Wärmespeicher zur Speicherung von Wärme aus der Dampfelektrolyse vorgesehen.

Wird zur Herstellung von Wasserstoff 44 bzw. 84 anstatt einer Wasserelektrolyse 41 bzw. 81 eine Wasserdampfelektrolyse 151 eingesetzt, wird in der Regel der gesamte in der Synthese erzeugte Dampf 61 bzw. 122 für die Erzeugung von Wasserstoff eingesetzt. Damit steht kein oder nur ein geringerer Anteil des Dampfes zur Wärmeversorgung für die $CO_2$-Desorption zur Verfügung.

In diesem Fall kann ein Teil des für die Schlusskühlung des Sauerstoffs 154 und des Wasserstoffs 150 aus der Wasserdampfelektrolyse abgeführten Wärmestromes 158 bzw. 156 für die Wärmebereitstellung 14 der $CO_2$-Desorption genutzt werden.

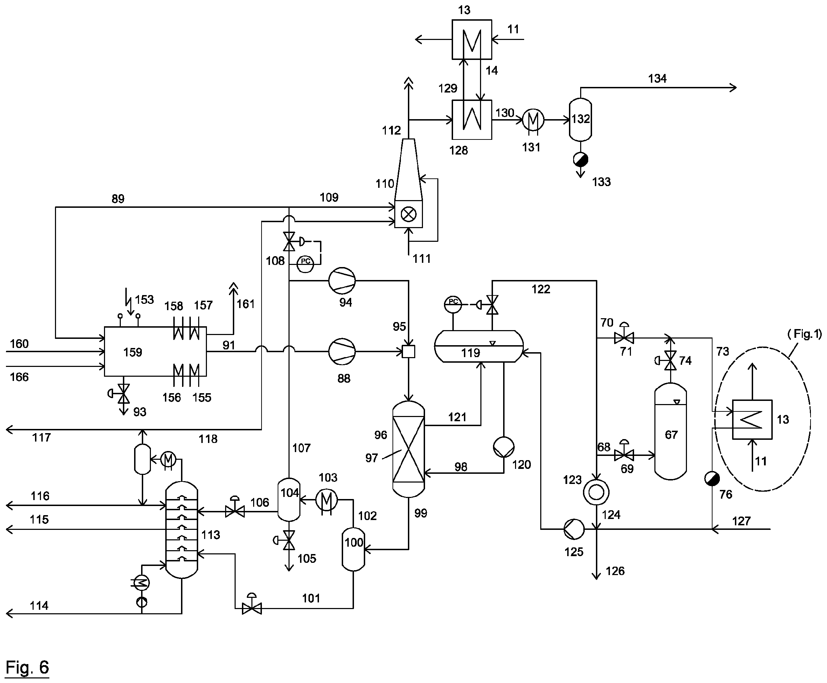

Co-Elektrolyse für Fischer-Tropsch-Synthese entsprechend **Fig. 3** und **Fig. 6:**

Zur Erzeugung eines kohlenstoffmonoxid- und wasserstoffhaltigen Synthesegases 91 aus Kohlenstoffdioxid 86, Wasser 80 oder Wasserdampf 152 (entsprechend Fig. 4) und Restgas 89 soll anstatt der Wasserelektrolyse 81 bzw. Wasserdampfelektrolyse 151 (entsprechend Fig. 4) und dem RWGS/ Reformierungsprozess 87 eine Co-Elektrolyse (Co-SOC) 159 (entsprechend Fig. 6) auf der Basis einer Solid Oxide Electrolysis Cell (SOEC) eingesetzt werden, in der durch regenerativ erzeugte Elektroenergie 153 Wasserdampf und Kohlenstoffdioxid eines Wasserdampf-Kohlenstoffdioxid-Gemisches 160 in wasserstoff- und kohlenstoffmonoxidhaltiges Synthesegas 91 und Sauerstoff 161 umgewandelt werden.

$$H_2O_D \rightarrow H_2 \ + 0,5 \ O_2 \qquad\qquad \Delta H_R = 241,8 \ kJ/mol$$

$$CO_2 \ \rightarrow CO + 0,5 \ O_2 \qquad\qquad \Delta H_R = 283,0 \ kJ/mol$$

Durch die hohe Temperatur in der Co-Elektrolyse 159 von ca. 850 °C wird auch das Restgas 89, was neben in der Synthese nicht umgesetzten Wasserstoff und Kohlenstoffmonoxid, auch Methan und andere nicht kondensierbare Kohlenwasserstoffe enthält, vor allem durch Reformierung der Kohlenwasserstoffe mit Wasserdampf mit zu Synthesegas aufgearbeitet.

$$C_xH_y + x \ H_2O \rightarrow x \ CO + (0,5y+x) \ H_2$$

Zur Bereitstellung des Wasserdampf-Kohlenstoffdioxid-Gemisches 160 für die Co-Elektrolyse 159 kann z.B. Kohlenstoffdioxid 86 aus der in Fig. 1 beschriebenen $CO_2$-Gewinnungsanlage und Wasserdampf 73 (entsprechend Fig. 3) entsprechend dem Stand der Technik aus der Synthesekühlung (beispielsweise aus der Druckschrift EP 2 491 998 A1) genutzt werden. Damit stände aber kein Wasserdampf mehr für die Wärmebereitstellung im Wärmeübertrager 13 der $CO_2$-Gewinnungsanlage zur Verfügung.

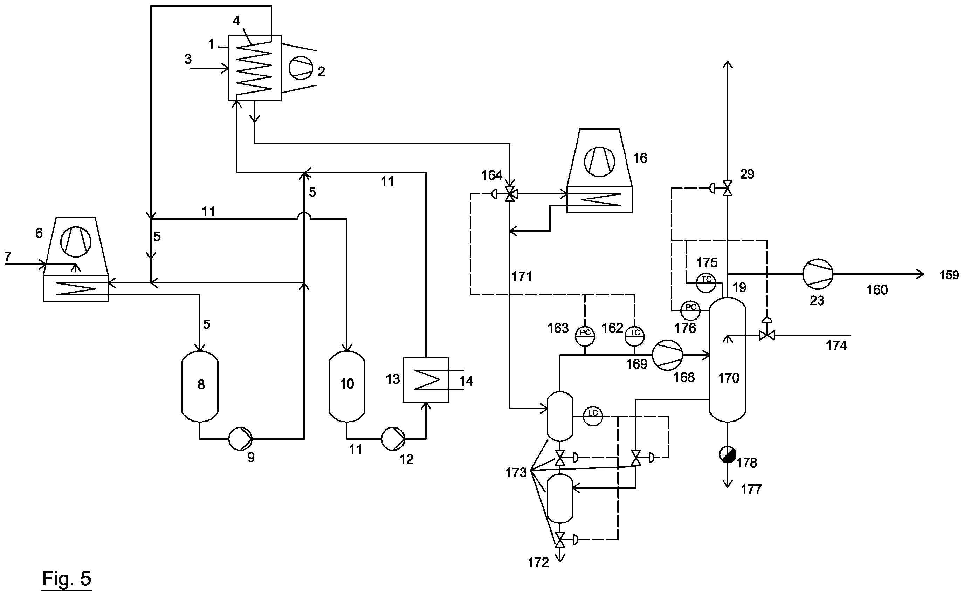

Es wird daher entsprechend **Fig. 5** vorgeschlagen, das im Regenerationsbetrieb mittels einer geeigneten Vakuumpumpe 168 aus dem Behälter 1 abgesaugte heiße Kohlenstoffdioxid-Wasserdampf-Gemisch mit einem Wasserdampfanteil je nach Luftfeuchtigkeit bei der jeweils vorigen $CO_2$ Adsorption von 0,5 bis 10, insbesondere 2 bis 5 kmol pro kmol Kohlenstoffdioxid im Kühler 16 nur soweit abzukühlen, dass im Kohlenstoffdioxidstrom 19 noch so viel Wasserdampf enthalten bleibt, wie für die nachfolgende Co-Elektrolyse 159 (entsprechend Fig. 6) zur Erzeugung eines Synthesegases 91 mit einem für die Synthese geforderten $H_2$-CO-Molverhältnisses erforderlich ist.

Die Vakuumpumpe 168 muss das nach dem Kühler 16 vorliegende, mit Wasserdampf gesättigte Gas 169 ohne Kondensation während des gesamten Verdichtungsprozesses komprimieren. Eine Zwischenkühlung ist dabei nur insoweit zulässig, als dass diese nicht zu einer Auskondensation von Wasser führt. In einer diesbezüglichen Ausführungsform ist sogar eine Überhitzung des wasserdampfhaltigen Gases durch den Verdichtungsprozess wünschenswert und von Vorteil, damit nach der Vakuumpumpe 168 im Puffer 170 und den Rohrleitungen bis zur Co-Elektrolyse 159 (Fig. 6) aufgrund von Wärmeverlusten nicht so schnell Kondensation auftreten kann.

Bei einer entsprechenden alternativen Ausgestaltung mittels einer zweistufigen Pumpanordnung könnte durchaus eine zumindest teilweise Zwischenkühlung stattfinden.

Eine Isolierung und eventuelle Begleitheizung der Ausrüstungen auf eine Temperatur größer als die Taupunkttemperatur des Wasserdampf-Kohlenstoffdioxid-Gemisches ist vorteilhaft, um Kondensation bis zur Co-Elektrolyse zu vermeiden.

Geeignete Vakuumpumpen sind beispielsweise trockenlaufende (ungekühlte) Kolbenverdichter und Schraubenverdichter.

Da sich das Kohlenstoffdioxid-Wasserdampf-Gemisch 171 nach der Kühlung im Kühler 16 am Taupunkt befindet, wird als Führungsgröße für die Regelung des Wasserdampfanteils im Kohlenstoffdioxid die Temperatur 162 in Abhängigkeit des Druckes 163 im Gasgemisch genutzt.

Am Taupunkt gilt:

$$p_D = p_s(t)$$

$$p_D + p_{CO2} = p_{ges}$$

$$r_D = p_D/p_{ges}$$

mit

$p_D$      Partialdruck Dampf im Gasgemisch
$p_s(t)$      Siededruck Dampf bei der Temperatur t
$r_D$      Molanteil bzw. Raumanteil Dampf im Gasgemisch
$p_{CO2}$      Partialdruck $CO_2$ im Gasgemisch
$p_{ges}$      Gesamtdruck

Geregelt werden beispielsweise die über eine Dreiwegearmatur 164 zum Kühler 16 geführte Kohlenstoffdioxid-Wasserdampf-Menge und/oder die Kühlleistung des Kühlers 16.

Dabei muss das im Kühler 16 auskondensierte Wasser 172 aus dem Gasstrom 171 abgetrennt und über ein Kondensatschleusensystem 173 vor der Vakuumpumpe 168 abgeschieden werden.

Eventuell vorhandene Überhitzungswärme im Gas 19 nach der Vakuumpumpe 168 kann durch Eindüsen von extern vorgewärmten Wasser 174 zur weiteren Aufsättigung des $CO_2$ mit Wasserdampf genutzt werden. Als Führungsgrößen für die Wassermenge dienen die Temperatur 175 und der Druck 176. Überschüssiges Wasser 177 wird über den Kondensatableiter 178 aus dem Behälter 170 abgeführt.

Sollte Dampf für die Co-Elektrolyse fehlen, da dieser nicht vollständig durch den Wasserdampfanteil im Kohlenstoffdioxid 19 und eventuell durch extern vorgewärmtes Wasser 174 abgedeckt werden kann, muss zusätzlich Dampf 166 aus einer externen Quelle der Co-Elektrolyse 159 zugeführt werden.

In einer alternativen Ausführung der Kohlenstoffdioxid-Gewinnungsanlage kann der Regenerationsbetrieb auch durch Spülung des Behälters 1 mit Dampf durchgeführt werden, entweder mit oder ohne gleichzeitig angewendetes Vakuum. Hierbei resultiert ebenfalls ein Kohlenstoffdioxid-Wasserdampf-Gemisch, welches aus dem Behälter 1 abgesaugt werden kann und welches im Kühler 16 soweit abgekühlt werden kann, dass im Kohlenstoffdioxidstrom 19 noch so viel Wasserdampf enthalten bleibt, wie für die nachfolgende Co-Elektrolyse 159 (entsprechend Fig. 6) zur Erzeugung eines Synthesegases 91 mit einem für die Synthese geforderten $H_2$-CO-Molverhältnisses erforderlich ist.

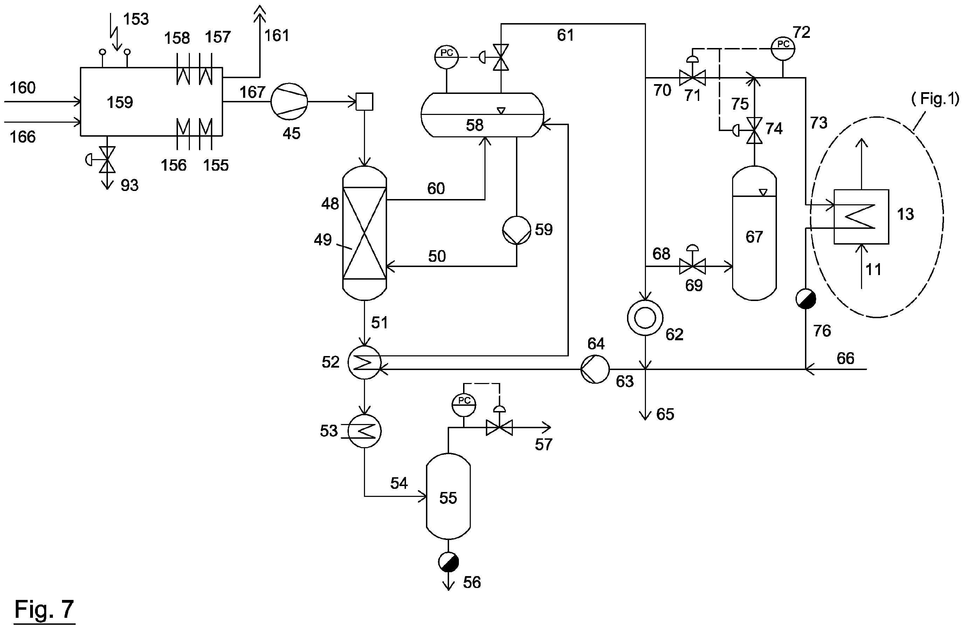

Co-Elektrolyse für Methanerzeugung (**Fig. 2** und **Fig. 7**):
In Fig. 7 ist eine Co-Elektrolyse 159 zur Erzeugung eines CO- und $H_2$-haltigen Synthesegases 167 und Sauerstoff 161 aus einem $H_2O$-$CO_2$-Gemisch 160 aus der $CO_2$-Gewinnung mit Hilfe von regenerativ erzeugter Elektroenergie 153 dargestellt.

Im Unterschied zu Fig. 2, wo Kohlenstoffdioxid 46 und Wasserstoff 44 aus der Elektrolyse 41 zur Methanerzeugung 57 nach folgender Reaktion

$$CO_2 + 4\ H_2 \rightarrow CH_4 + 2\ H_2O \qquad \Delta H_R = -165,1\ kJ/mol$$

eingesetzt wird, erfolgt in Fig. 7 die Umwandlung von Kohlenstoffmonoxid und Wasserstoff aus dem Synthesegas 167 nach folgender Reaktion zu Methan:

$$CO + 3\ H_2 \rightarrow CH_4 + H_2O \qquad \Delta H_R = -206,2\ kJ/mol$$

Die Erzeugung von Methan aus Kohlenstoffmonoxid und Wasserstoff ist Stand der Technik.

Insgesamt ergeben sich für die diesseitige Erfindung noch weitere Vorteile, nämlich:
Durch die Reduzierung der Kühlung des desorbierten Kohlenstoffdioxids und damit den Erhalt des Wasserdampfes aus der Luftfeuchtigkeit im Kohlenstoffdioxid der $CO_2$-Erzeugungsanlage kann ein Teil der zur Desorption von Kohlenstoffdioxid und Wasser (Verdampfung) eingesetzten Wärme hauptsächlich in Form von Wasserdampf erhalten bleiben und für die Erzeugung von Wasserstoff gemeinsam mit Kohlenstoffmonoxid aus Kohlenstoffdioxid in einer Co-Elektrolyse auf SOEC-Basis genutzt werden.

Es wird dabei durch die Kühlung anfallendes Abwasser (Kondensat) und der Einsatz von zusätzlichem Wasser für die Erzeugung von Wasserstoff in der Elektrolyse vermieden.

Weiterhin wird zusätzliche Wärme zur Verdampfung von Wasser in einer Wasserelektrolyse, die durch Elektroenergie aufgebracht werden muss, oder für die externe Verdampfung von Wasser vor einer Wasserdampfelektrolyse eingespart, wodurch die Effizienz des Gesamtprozesses steigt.

Der Einsatz einer Co-Elektrolyse anstatt einer Elektrolyse und einem RWGS-Prozess zur Erzeugung von Synthesegas reduziert die notwendigen Verfahrensschritte zur Erzeugung von Synthesegas auf einen Elektrolyseschritt, was zur Einsparung von Ausrüstungen und damit zur Reduzierung der Investitionskosten führt.

Durch die in situ Erzeugung der Synthesegaskomponenten CO und $H_2$ in der Co-Elektrolyse bei einer Temperatur werden Zwischenabkühlungen vermieden und Wärmeverluste reduziert, was zur Wirkungsgradsteigerung der Synthesegaserzeugung und weiterer Erhöhung der Effizienz des Gesamtprozesses und damit zur Reduzierung von Betriebskosten führt.

Weiteres Ausführungsbeispiel:

Nachfolgend erfolgt die Beschreibung der Erfindung anhand eines konkreten Ausführungsbeispiels unter Hinzunahme der nachfolgenden Diagramme 1 bis 6 und der zuvor erläuterten Figuren 1 bis 4:
Eine Anlage zur Gewinnung von Kohlenstoffdioxid aus der Umgebungsluft soll mit einer Anlage zur Erzeugung von Methan aus Kohlenstoffdioxid und Wasserstoff, hergestellt mittels Wasserelektrolyse und regenerativ erzeugtem Strom, gekoppelt werden.

Die $CO_2$-Gewinnungsanlage hat eine Jahresleistung von 1.000 t $CO_2$/a, das entspricht bei einer jährlichen Ausnutzung der Anlage von 8.000 Stunden einer Leistung von 125 kg $CO_2$/h.

Der $CO_2$-Bedarf der Methanisierungsanlage soll je nach Anfall von regenerativ erzeugtem Überschussstrom im Elektroenergieverteilernetz zwischen 50 kg $CO_2$/h und 175 kg $CO_2$/h schwanken.

Zum Ausgleich der Lastschwankungen werden zwei $CO_2$-Druckspeicher und ein Ruthsspeicher zur Speicherung des Dampfes aus der Synthese eingesetzt.

Die Größen der Speicher sind:

| | | |
|---|---|---|
| $CO_2$-Speicher (25): | 10 m$^3$ | Arbeitsdruck: 20...25 bara |
| $CO_2$-Speicher (31): | 10 m$^3$ | Arbeitsdruck: 20...50 bara |
| Ruthsspeicher (67): | 12,7 m$^3$ | Arbeitsdruck: 2...25 bara |

Die Anlagenparameter, nämlich Stellung der Armaturen 29, 30, 37, Zustand der Verdichter 23 und 32, Druck in den Behältern 25 und 31, Überschussdampfmenge, Druck im Ruthsspeicher 67 sowie im Ruthsspeicher 67 gespeicherte Energie werden in den Diagrammen 1 bis 6 als Funktion der Zeit dargestellt.

Im Nennlastfall beider Anlagen (125 kg $CO_2$/h) beträgt der Dampfbedarf der $CO_2$-Gewinnungsanlage 338 kg/h bzw. die Dampferzeugung der Methananlage beträgt 243 kg/h, d.h. die Methanerzeugungsanlage kann den Dampfbedarf der $CO_2$-Gewinnungsanlage nur zu ca. 72 % decken.

Im Folgenden wird davon ausgegangen, dass eine konstante Grundversorgung mit Dampf für die $CO_2$-Gewinnungsanalge von 95 kg/h durch einen Fremdanbieter erfolgt. Damit deckt im Nennlastfall beider Anlagen der Zusatzdampf aus der Methanerzeugung den Dampfbedarf der $CO_2$-Erzeugung.

Zum Zeitpunkt 0 laufen beide Anlagen im Nennlastbetrieb (125 kg $CO_2$/h). Der Zustand der Anlage ist:

| | | |
|---|---|---|
| - produzierte $CO_2$-Menge $CO_2$-Gewinnungsanlage: | | 125 kg/h |
| - $CO_2$-Bedarf Methanerzeugungsanlage: | | 125 kg/h |
| - Stellung der Armaturen | 29: | geschlossen |
| | 30: | geöffnet |
| | 37: | geschlossen |
| - Zustand der Verdichter | 23: | in Betrieb |
| | 32: | nicht in Betrieb |
| - Druck in den Behältern | 25: | 25 bara |
| | 31: | 50 bara |
| - Überschussdampfmenge: | | 0 kg/h |
| - Druck im Ruthsspeicher | 67: | 2 bara |

- im Ruthsspeicher 67 gespeicherte Energie: (Restwärme, nicht nutzbar)    1.060,5 kWh

Die Gaspuffer 25 und 31 sind beide geladen. Das produzierte $CO_2$ wird mit dem Verdichter 23 durch den Behälter 25 quasi durchgeschoben und der Methanisierung (Fig. 2) zur Verfügung gestellt.

Der Druck im Ruthsspeicher ist auf Abgabedruck abgesunken. Der in der Methanisierung erzeugte Dampf wird sofort der $CO_2$-Erzeugung zur Verfügung gestellt und deckt dort den aktuellen Dampfbedarf.

Vom Zeitpunkt 0 bis zum Zeitpunkt 1 wird für 2,38 Stunden die Leistung der Methanisierung auf 175 kg $CO_2$/h angehoben.

Der Zustand der Anlage ist dann:

| | | |
|---|---|---|
| - produzierte $CO_2$-Menge $CO_2$-Gewinnungsanlage: | | 125 kg/h |
| - $CO_2$-Bedarf Methanerzeugungsanlage: | | 175 kg/h |
| - Stellung der Armaturen | 29: | geschlossen |
| | 30: | geöffnet |
| | 37: | geschlossen |
| - Zustand der Verdichter | 23: | in Betrieb |
| | 32: | nicht in Betrieb |
| - Druck in den Behältern | 25: | 20 bara |
| | 31: | 50 bara |
| - Überschussdampfmenge: | | 97,2 kg/h |
| - Druck im Ruthsspeicher | 67: | 3,23 bara |
| - im Ruthsspeicher 67 gespeicherte Energie: | | 1.240,5 kWh |

Der Druck im Gaspuffer 25 ist in Folge des höheren Bedarfs auf 20 bara abgesunken. Die erhöhte Dampfproduktion von 97,2 kg/h führt zu einem Druckanstieg im Ruthsspeicher auf 3,23 bara.

Vom Zeitpunkt 1 bis zum Zeitpunkt 2 wird für 17,03 Stunden (absolut 19,41 Stunden) die Leistung der Methanisierung bei 175 kg $CO_2$/h konstant gehalten.

Der Zustand der Anlage ist:

| | | |
|---|---|---|
| - produzierte $CO_2$-Menge $CO_2$-Gewinnungsanlage: | | 125 kg/h |
| - $CO_2$-Bedarf Methanerzeugungsanlage: | | 175 kg/h |
| - Stellung der Armaturen | 29: | geschlossen |

(fortgesetzt)

|  |  | 30: | geöffnet |
|---|---|---|---|
|  |  | 37: | geöffnet |
| - Zustand der Verdichter |  | 23: | in Betrieb |
|  |  | 32: | nicht in Betrieb |
| - Druck in den Behältern |  | 25: | 20 bara |
|  |  | 31: | 20 bara |
| - Überschussdampfmenge: |  |  | 97,2 kg/h |
| - Druck im Ruthsspeicher |  | 67: | 25 bara |
| - im Ruthsspeicher 67 gespeicherte Energie: |  |  | 2.528,4 kWh |

Der Druck im Gaspuffer 31 ist in Folge des höheren Bedarfs ebenfalls auf 20 bara abgesunken. Die erhöhte Dampfproduktion von 97,2 kg/h führt zu einem Druckanstieg im Ruthsspeicher auf 25 bara. Der Ruthsspeicher hat seinen oberen Druckwert erreicht.

Vom Zeitpunkt 2 bis zum Zeitpunkt 3 wird für 1,59 Stunden (absolut 21,0 Stunden) die Leistung der Methanisierung auf 50 kg $CO_2$/h abgesenkt.

Der Zustand der Anlage ist:

| - produzierte $CO_2$-Menge $CO_2$-Gewinnungsanlage: |  | 125 kg/h |
|---|---|---|
| - $CO_2$-Bedarf Methanerzeugungsanlage: |  | 50 kg/h |
| - Stellung der Armaturen | 29: | geschlossen |
|  | 30: | geöffnet |
|  | 37: | geschlossen |
| - Zustand der Verdichter | 23: | in Betrieb |
|  | 32: | nicht in Betrieb |
| - Druck in den Behältern | 25: | 25 bara |
|  | 31: | 20 bara |
| - Überschussdampfmenge: |  | -145,8 kg/h |
| - Druck im Ruthsspeicher | 67: | 20,6 bara |
| - im Ruthsspeicher 67 gespeicherte Energie: |  | 2.348,6 kWh |

Der Druck im Gaspuffer 25 ist aufgrund des geringeren $CO_2$-Bedarfs in der Methanisierung wieder auf 25 bara angestiegen. Der Druck im Gaspuffer 31 beträgt noch 20 bara. Die geringere Dampfproduktion in der Methanisierung führt zur Dampfentnahme aus dem Ruthsspeicher von 145,8 kg/h. Dadurch sinkt der Druck auf 20,6 bara ab.

Vom Zeitpunkt 3 bis zum Zeitpunkt 4 bleibt für 11,35 Stunden (absolut 32,35 Stunden) die Leistung der Methanisierung bei 50 kg $CO_2$/h konstant.

Der Zustand der Anlage ist:

| - produzierte $CO_2$-Menge $CO_2$-Gewinnungsanlage: |  | 125 kg/h |
|---|---|---|
| - $CO_2$-Bedarf Methanerzeugungsanlage: |  | 50 kg/h |
| - Stellung der Armaturen | 29: | geschlossen |
|  | 30: | geöffnet |
|  | 37: | geschlossen |
| - Zustand der Verdichter | 23: | in Betrieb |
|  | 32: | in Betrieb |
| - Druck in den Behältern | 25: | 25 bara |
|  | 31: | 50 bara |
| - Überschussdampfmenge: |  | -145,8 kg/h |

(fortgesetzt)

| | | | |
|---|---|---|---|
| - Druck im Ruthsspeicher | | 67: | 2,3 bara |
| - im Ruthsspeicher 67 gespeicherte Energie: | | | 1.101,3 kWh |

Der Verdichter 32 puffert den Behälter 31 mit der Überschuss- $CO_2$-Menge wieder auf 50 bara auf.

Die geringere Dampfproduktion in der Methanisierung führt zur weiteren Dampfentnahme aus dem Ruthsspeicher von 145,8 kg/h. Dadurch sinkt der Druck auf 2,3 bara ab.

Damit ist der Ausgangszustand wieder erreicht und der Zyklus kann von vorn beginnen. Der geringfügig höhere Druck im Ruthsspeicher ist darauf zurückzuführen, dass der Dampf mit einer spezifisch höheren Enthalpie zugeführt wird als abgeführt wird. Durch praktisch auftretende Wärmeverluste und/ oder zusätzliche Kühler wird dieser Effekt ausgeglichen.

Die Zeitabschnitte wurden hier so gewählt, dass die Speicher jeweils vollständig geladen und anschließend vollständig entladen werden. Praktisch werden die Zeitzyklen entsprechend des Überschussstromanfalls anders aussehen, so dass die Speicher auch nur Zwischenzustände erreichen.

Beim Einsatz einer Fischer-Tropsch-Synthese wird ähnlich so viel Dampf wie bei der Methanisierung erzeugt.

Wird die Wärme der Wasserdampfelektrolyse, auch zusätzlich, genutzt, kann diese ca. 35 % des Wärmebedarfs der Desorption decken.

Diagramm 1

## Zustand (1= Betrieb, 0= nicht Betrieb) der Verdichter als Funktion der Zeit

Diagramm 2

## Zustand der Armaturen (1= offen, 0= geschlossen) als Funktion der Zeit

Diagramm 3

**Diagramm 4**

**Diagramm 5**

**Im Dampfpuffer gespeicherte Energie als Funktion der Zeit**

Diagramm 6

Bezugszeichenliste

| | | | | | |
|---|---|---|---|---|---|
| Fig. 1 | Kohlenstoffdioxidgewinnungsanlage | | 31 | Langzeitspeicher |
| Fig. 2 | Methansyntheseanlage | | 32 | Verdichter |
| Fig. 3 | Fischer-Tropsch-Syntheseanlage | | 33 | Kühler |
| Fig. 4 | Wasserdampfelektrolyse (SOEC) | | 34 | Kondensat |
| 1 | Behälter mit Adsorbermaterial | | 35 | Kondensatableiter |
| 2 | Gebläse | | 36 | Druck |
| 3 | Umgebungsluft | | 37 | Regelventil |
| 4 | Wärmeübertragerfläche | | 40 | deionisiertes Wasser |
| 5 | Kühlflüssigkeit | | 41 | Elektrolyseapparat |
| 6 | Kühlturm | | 42 | Elektroenergie |
| 7 | Wasser | | 43 | Sauerstoff |
| 8 | Kühlmittelbehälter | | 44 | Wasserstoff |
| 9 | Kühlmittelpumpe | | 45 | Verdichter |
| 10 | Heizmittelbehälter | | 46 | Kohlenstoffdioxid |
| 11 | Heizflüssigkeit | | 47 | Mischgas |
| 12 | Heizmittelpumpe | | 48 | Synthesereaktor |
| 13 | Wärmeübertrager | | 49 | Katalysator |
| 14 | Wärmequelle / Wärme | | 50 | Siedewasserkreislauf |
| 15 | Flüssigkeitsringverdichter | | 51 | Reaktionsgas |
| 16 | Kühlturm | | 52 | Wärmeübertrager |
| 17 | Kondensat | | 53 | Kühler |
| 18 | Trennbehälter | | 54 | Gasgemisch |
| 19 | Kohlenstoffdioxid | | 55 | Abscheider |
| 20 | Kondensat | | 56 | Reaktionswasser |
| 21 | Kühlwasser | | 57 | Methan |

(fortgesetzt)

| 22 | Kühler | 58 | Dampftrommel |
|---|---|---|---|
| 23 | Verdichter | 59 | Pumpe |
| 24 | Gaskühlung | 60 | dampfhaltiges Siedewasser |
| 25 | Gaspuffer | 61 | Sattdampf |
| 26 | Wasserkondensat | 62 | Wärmesenke |
| 27 | Kondensatableiter | 63 | Speisewasser |
| 28 | Behälterdruck | 64 | Pumpe |
| 29 | Regelventil | 65 | Dampfkondensatverlust |
| 30 | Regelventil | 66 | Speisewasser |
| 70 | Dampf | 109 | Gas |
| 71 | Drosselventil | 110 | Verbrennungseinrichtung |
| 72 | Druck | 111 | Luft |
| 73 | Heizdampf | 112 | Rauchgas |
| 74 | Drosselventil | 113 | Produktaufbereitung |
| 75 | Differenzdampfmenge | 114 | Wachs |
| 76 | Dampfkondensat | 115 | Diesel |
| 80 | deionisiertes Wasser | 116 | Naphtha |
| 81 | Elektrolyseapparat | 117 | leichtes Kohlenwasserstoffgas |
| 82 | Elektroenergie | 118 | Restgas |
| 83 | Sauerstoff | 119 | Dampftrommel |
| 84 | Wasserstoff | 120 | Pumpe |
| 85 | Verdichter | 121 | Siedewasser |
| 86 | Kohlenstoffdioxid | 122 | Sattdampf |
| 87 | RWGS/ Reformierungs-Prozess | 123 | Wärmesenke |
| 88 | Verdichter | 124 | Dampfkondensat |
| 89 | Restgas | 125 | Pumpe |
| 90 | Elektroenergie | 126 | Dampfkondensatverlust |
| 91 | Synthesegas | 127 | Speisewasser |
| 92 | Kühlung | 128 | Wärmeübertrager |
| 93 | Kondensat | 129 | Wärme |
| 94 | Verdichter | 130 | Rauchgas |
| 95 | Kreislaufgas | 131 | Kühler |
| 96 | Fischer-Tropsch-Reaktor | 132 | Trennbehälter |
| 97 | Katalysator | 133 | Abwasser |
| 98 | Siedewasserkreislauf | 134 | Gasstrom |
| 99 | Reaktionsprodukt | 150 | Wasserstoff |
| 100 | Hochtemperaturabscheider | 151 | Wasserdampf-Elektrolyse |
| 101 | flüssiger Kohlenwasserstoff | 152 | Wasserdampf |
| 102 | kohlenwasserstoffhaltiges Gas | 153 | Elektroenergie |
| 103 | Kühler | 154 | Sauerstoff |
| 104 | Trennbehälter | 155 | überschüssige Wärme |
| 105 | Reaktionswasser | 156 | überschüssige Wärme |
| 106 | flüssiger Kohlenwasserstoff | 157 | überschüssige Wärme |
| 107 | Restgas | 158 | überschüssige Wärme |
| 108 | Teilstrom Restgas | 159 | Co-Elektrolyse |
| 160 | $CO_2$-Dampf-Gemisch | 170 | Behälter |
| 161 | Sauerstoff | 171 | wasserdampfgesättigtes $CO_2$ mit Kondensat |
| 162 | Temperatur | | |
| 163 | Druck | 172 | Kondensat |
| 164 | Armatur | 173 | Schleusensystem |
| 165 | Armatur | 174 | vorgewärmtes Zusatzwasser |

(fortgesetzt)

| | | | |
|---|---|---|---|
| 166 | Zusatzdampf | 175 | Temperatur |
| 167 | Synthesegas | 176 | Druck |
| 168 | Vakuumpumpe | 177 | Kondensat, Überschusswasser |
| 169 | wasserdampfgesättigtes $CO_2$ | 178 | Kondensatableiter |

**Patentansprüche**

1. Herstellungsverfahren von synthetisch hergestelltem Methan (57) / gasförmigen und/oder flüssigen Kohlenwasserstoffen (114, 115, 116, 117),

   wobei hierzu Wasserstoff (44, 84, 150) aus einer mit Hilfe von regenerativ erzeugter Elektroenergie betriebenen Elektrolyseanordnung (41, 81, 151) und Kohlenstoffdioxid (19, 46, 86) in einer Methan- (Fig. 2 - 48) oder Fischer-Tropsch-Synthese (Fig. 3 - 96) oder einer anderen Kohlenwasserstoffsynthese, kombiniert werden,
   **dadurch gekennzeichnet, dass**
   das Kohlenstoffdioxid (19, 46, 86) aus einem Umgebungsluftstrom (3, 134) mittels einer Kohlenstoffdioxidgewinnungsanlage (Fig. 1) hergestellt wird, wobei das Kohlenstoffdioxid (19, 46, 86) aus dem Umgebungsluftstrom (3, 134) in der Kohlenstoffdioxidgewinnungsanlage (Fig. 1) zunächst mittels eines Adsorptionsmaterials adsorbiert wird und mittels Temperatur-Vakuum-Wechselverfahren gewonnen wird,
   wobei
   zur Umkehrung des Adsorptionsprozesses und damit zur Freigabe des adsorbierten Kohlenstoffdioxides (19, 46, 86) die thermische Desorption durchgeführt wird, wobei hierzu Wärme (14 über 11) in das Adsorbermaterial (1) eingebracht wird und diese Wärme wenigstens teilweise aus der exothermen Methan- (Fig. 2 - 48) oder Fischer-Tropsch-Synthese (Fig. 3 - 96) oder aus einer anderen geeigneten Kohlenwasserstoffsynthese und/oder der Elektrolyseanordnung (41, 81, 151) entnommen wird.

2. Herstellungsverfahren nach Anspruch 1,
   **dadurch gekennzeichnet, dass**
   die Wärme (14) aus in dem Syntheseprozess anfallenden Dampf (61, 70, 122) entnommen wird.

3. Herstellungsverfahren nach einem der Ansprüche 1 oder 2,
   **dadurch gekennzeichnet, dass**
   die Elektrolyseanordnung (41, 81, 151) eine mit regenerativ erzeugter Elektroenergie arbeitende Wasserdampfelektrolyse (151) (SOEC) ist und anfallende Wärme aus Kühlung (155, 156, 157, 158) eines Sauerstoff- und/oder Wasserstoffgasstroms zur Umkehrung (14 über 1 1) des Adsorptionsprozesses entnommen wird.

4. Herstellungsverfahren nach einem der vorangehenden Ansprüche,
   **dadurch gekennzeichnet, dass**
   im Herstellungsverfahren anfallendes überschüssiges und/oder nicht verwendbares Restgas (109) verbrannt (110) wird und ein sich bildendes Rauchgas (1 12) stofflich (130, 134) und/oder energetisch (129) genutzt wird, wobei hierzu die Wärme (129) des Rauchgases zur Umkehrung des Adsorptionsprozesses (14 über 11) genutzt wird und/oder das Rauchgas (1 12) als Zusatz (134) in dem Umgebungsluftstrom (3) eingesetzt wird.

5. Herstellungsverfahren nach einem der Ansprüche 1 bis 4,
   **dadurch gekennzeichnet, dass**
   Niedertemperaturabwärme mit Temperaturen unterhalb von 80°C zur Regeneration des adsorbierten Wassers in der Kohlenstoffgewinnungsanlage (Fig. 1) eingesetzt wird.

6. Herstellungsverfahren nach einem der vorangehenden Ansprüche,
   **dadurch gekennzeichnet, dass**
   ein Wärmepumpenprozess zur Erhöhung der Temperatur von Wärme aus dem Syntheseprozess (61, 122) und/oder Restgasnutzung (129) und/oder Kühlung in der Elektrolyse (155, 156, 157, 158) und/oder weiterer Prozessabwärme verwendet wird.

7. Herstellungsverfahren nach einem der vorangehenden Ansprüche,
   **dadurch gekennzeichnet, dass**

die Kohlenstoffdioxidgewinnungsanlage (Fig. 1) kontinuierlich betrieben wird, wobei der Kohlenstoffdioxidbedarf des Syntheseprozesses (Fig. 2, Fig. 3) aufgrund von Schwankungen von zur Verfügung stehender regenerativ erzeugter Elektroenergie (42, 82, 90, 153) diskontinuierlich erfolgt und das gewonnene Kohlenstoffdioxid (19, 46, 86) in einem Pufferspeicher (25, 31) zwischengespeichert wird,

wobei bevorzugt das Zwischenpuffern des Kohlenstoffdioxides (19, 46, 86) in einem Kurzzeitspeicher (25) und einem parallel dazu geschalteten Langzeitspeicher (31) mit einem höheren Druck arbeitend erfolgt, wobei das Langzeitzwischenpuffern über ein Verflüssigen des Kohlenstoffdioxides (19, 46, 86) erfolgt.

8. Herstellungsverfahren nach einem der vorangehenden Ansprüche,
**dadurch gekennzeichnet, dass**
der Syntheseprozess (Fig. 2, Fig. 3) aufgrund von Schwankungen von zur Verfügung stehender regenerativ erzeugter Elektroenergie (42, 82, 92, 153) diskontinuierlich erfolgt und die nutzbare Wärme (61, 122) für die Kohlenstoffdioxidgewinnungsanlage (Fig. 1) in einem Pufferspeicher (67) zwischengespeichert wird.

9. Herstellungsverfahren nach einem der vorangehenden Ansprüche,
**dadurch gekennzeichnet, dass**
bei Betrieb der Kohlenstoffdioxidgewinnungsanlage (Fig. 1) gewonnenes Wasser aus der Atmosphäre für die Elektrolyse (41, 81, 151) eingesetzt wird.

10. Herstellungsverfahren nach einem der vorangehenden Ansprüche,
**dadurch gekennzeichnet, dass**
zusätzlich zu Wasserstoff (44, 84, 150) noch Kohlenmonoxid aus Kohlenstoffdioxid (19, 46, 86) mit Hilfe der mit regenerativ erzeugter Elektroenergie betriebenen Elektrolyseanordnung (41, 81, 151, 159) gewonnen wird.

11. Herstellungsverfahren nach Anspruch 10,
**dadurch gekennzeichnet, dass**
die Elektrolyseanordnung (41, 81, 151, 159) eine Wasserdampfelektrolyse (151) ist, wobei der Dampf direkt aus dem während des Regenerationsbetriebes der $CO_2$-Gewinnungsanlage aus dem Behälter (1) abgesaugten Kohlenstoffdioxid-Wasserdampf-Gemisch gewonnen wird, ohne dass der Wasserdampf zwischenzeitlich kondensiert wird.

12. Herstellungsanlage zur Produktion von synthetisch hergestelltem Methan (57) / gasförmigen und/oder flüssigen Kohlenwasserstoffen (1 14, 115, 116, 117), insbesondere zur Durchführung des Herstellungsverfahrens nach einem der vorangehenden Ansprüche, mit:

- einer mit regenerativ erzeugter Elektroenergie (42, 82, 153) betriebenen Elektrolyseanordnung (41, 81, 151) zur Herstellung von Wasserstoff (44, 84, 150),
- einer Kohlenstoffdioxidgewinnungsanlage (Fig. 1) zur Herstellung von Kohlenstoffdioxid (19, 46, 86) aus einem Umgebungsluftstrom (3, 134), wobei die Kohlenstoffdioxidgewinnungsanlage (Fig. 1) mit einem Adsorbermaterial arbeitet, das mittels Temperatur-Vakuum-Wechselverfahren das gebundene Kohlenstoffdioxid wieder abgibt und
- einer Methan- (Fig. 2) oder Fischer-Tropsch-Synthese (Fig. 3) oder einer anderen Kohlenwasserstoffsynthese zur Synthetisierung von Wasserstoff (44, 84, 150) und Kohlenstoffdioxid (19, 46, 86) zu Methan (57) / gasförmigen und/oder flüssigen Kohlenwasserstoffen (114, 1 15, 1 16, 1 17),

wobei
Wärmezufuhrmittel (4, 10, 11, 12, 13, 14, 128) und/oder Wärmespeicher (67) zur Zuführung und/oder Zwischenspeicherung von Wärme von der Synthese (61, 122) und/oder Wärme der Elektrolyseanordnung (155, 156, 157, 158) und/oder Wärme einer Verbrennungseinrichtung für Restgase (129) aus dem Syntheseprozess zur Kohlenstoffdioxidgewinnungsanlage (Fig. 1 über 14) vorgesehen sind, wobei die Wärme (14 über 11) genutzt wird, um die thermische Desorption durchzuführen.

13. Herstellungsanlage nach Anspruch 12,
**dadurch gekennzeichnet, dass**
an dem Methan- (48) oder Fischer-Tropsch-Synthesereaktor (96) oder dem Reaktor einer anderen geeigneten Kohlenwasserstoffsynthese zur Abführung der Reaktionswärme eine Dampftrommel (58, 119) zur Trennung des den Synthesereaktor (48, 96) verlassenden dampfhaltigen Siedewassers (60, 121) in Siedewasser (50, 98) und Sattdampf (61, 122) vorgesehen ist, wobei Zufuhrmittel für den Sattdampf (61, 122) zu einem Wärmeüberträger (14) eines Heizmittelkreislaufes (11) der Kohlenstoffdioxidgewinnungsanlage (Fig. 1) vorgesehen sind.

14. Herstellungsanlage nach einem der Ansprüche 12 oder 13,
**dadurch gekennzeichnet, dass**
die Elektrolyseanordnung (41, 81, 151, 159) eine Wasserdampfelektrolyse (151) (SOEC) ist, wobei Zufuhrmittel (155, 156, 157, 158) zum Transport von Wärme aus einer Kühlung des gebildeten Wasserstoff- (154) /Sauerstoffstroms (150) zu einem Wärmeüberträger (14) eines Heizmittelkreislaufes (11) der Kohlenstoffdioxidgewinnungsanlage (Fig. 1) vorgesehen sind.

15. Herstellungsanlage nach einem der Ansprüche 12 bis 14,
**dadurch gekennzeichnet, dass**

eine Wärmepumpenanordnung zur Erhöhung der Temperatur von nutzbarer Abwärme aus der Elektrolyseanordnung (155, 156, 157, 158), dem Synthesereaktor (61, 122) und/oder Verbrennungseinrichtung (129) für Restgase vorgesehen ist
und/oder
Speichermittel (25, 31) zur Speicherung und Zuführung zur Methan- (Fig. 2) oder Fischer-Tropsch-Synthese (Fig. 3) oder zu einer anderen geeigneten Kohlenwasserstoffsynthese des in der Kohlenstoffdioxidgewinnungsanlage (Fig. 1) kontinuierlich produzierten Kohlenstoffdioxids (19, 46, 86) vorgesehen sind.

**Claims**

1. Production process of synthetically produced methane (57)/gaseous and/or liquid hydrocarbons (114, 115, 116, 117),

wherein hydrogen (44, 84, 150) from an electrolysis arrangement (41, 81, 151) operated with the aid of regeneratively generated electrical energy and carbon dioxide (19, 46, 86) are combined in a methane synthesis (Fig. 2 - 48) or Fischer-Tropsch synthesis (Fig. 3 - 96) or another hydrocarbon synthesis,
**characterised in that**
the carbon dioxide (19, 46, 86) is produced from an ambient air stream (3, 134) by means of a carbon dioxide recovery plant (Fig. 1), wherein the carbon dioxide (19, 46, 86) is first adsorbed from the ambient air stream (3, 134) in the carbon dioxide recovery plant (Fig. 1) by means of an adsorption material and is recovered by means of a temperature-vacuum alternating process,
whereby
a thermal desorption is carried out to reverse the adsorption process and thus to release the adsorbed carbon dioxide (19, 46, 86), heat (14 via 11) being introduced into the adsorber material (1) for this purpose and this heat being taken at least partially from the exothermic methane synthesis (Fig. 2 - 48) or Fischer-Tropsch synthesis (Fig. 3 - 96) or from another suitable hydrocarbon synthesis and/or the electrolysis arrangement (41, 81, 151).

2. Manufacturing process according to claim 1, **characterised in that** the heat (14) is extracted from vapour (61, 70, 122) produced in the synthesis process.

3. Manufacturing process according to one of claims 1 or 2, **characterised in that** the electrolysis arrangement (41, 81, 151) is a steam electrolysis (151) (SOEC) operating with regeneratively generated electrical energy and the heat generated from Cooling (155, 156, 157, 158) of an oxygen gas stream and/or hydrogen gas stream for the reversal (14 over 11) of the adsorption process.

4. Manufacturing process according to one of the preceding claims, **characterised in that**
excess and/or unusable residual gas (109) arising in the manufacturing process is combusted (110) and a flue gas (1 12) formed is utilised for material (130, 134) and/or energy (129) purposes, wherein the heat (129) of the flue gas being used for this purpose to reverse the adsorption process (14 via 11) and/or the flue gas (112) being used as an additive (134) in the ambient air flow (3).

5. Manufacturing process according to one of claims 1 to 4,
**characterised in that**
Low-temperature waste heat with temperatures below 80°C is used to regenerate the adsorbed water in the carbon extraction plant (Fig. 1).

6. Manufacturing process according to one of the preceding claims, **characterised in that** a heat pump process is used to increase the temperature of heat from the synthesis process (61, 122) and/or residual gas utilisation (129) and/or cooling in the electrolysis (155, 156, 157, 158) and/or further process waste heat.

7. Manufacturing process according to one of the preceding claims, **characterised in that**

the carbon dioxide production plant (Fig. 1) is operated continuously, wherein the carbon dioxide requirement of the synthesis process (Fig. 2, Fig. 3) being intermittent due to fluctuations in the available regeneratively generated electrical energy (42, 82, 90, 153) and the carbon dioxide produced (19, 46, 86) being stored temporarily in a buffer store (25, 31), wherein the intermediate buffering of the carbon dioxide (19, 46, 86) preferably takes place in a short-term accumulator (25) and a long-term accumulator (31) connected in parallel thereto and operating at a higher pressure, wherein the long-term intermediate buffering takes place by liquefying the carbon dioxide (19, 46, 86).

8. Manufacturing process according to one of the preceding claims, **characterised in that** the synthesis process (Fig. 2, Fig. 3) takes place intermittently due to fluctuations in the available regeneratively generated electrical energy (42, 82, 92, 153) and the usable heat (61, 122) for the carbon dioxide production plant (Fig 1) is temporarily stored in a buffer store (67).

9. Manufacturing process according to one of the preceding claims, **characterised in that** water extracted from the atmosphere during operation of the carbon dioxide extraction plant (Fig. 1) is used for electrolysis (41, 81, 151).

10. Manufacturing process according to one of the preceding claims, **characterised in that** in addition to hydrogen (44, 84, 150), carbon monoxide is also obtained from carbon dioxide (19, 46, 86) with the aid of the electrolysis arrangement (41, 81, 151, 159) powered by regeneratively generated electrical energy.

11. Manufacturing process according to claim 10, **characterised in that** the electrolysis arrangement (41, 81, 151, 159) is a water steam electrolysis (151), wherein the steam is obtained directly from the carbon dioxide/water vapour mixture extracted from the container (1) during the regeneration operation of the $CO_2$ extraction plant, without the water vapour being condensed in the meantime.

12. Production plant for the production of synthetically produced methane (57) / gaseous and/or liquid hydrocarbons (114, 115, 116, 117),

in particular for the carrying out the production process according to of one of the preceding claims, comprising

- one electrolysis arrangement (41, 81, 151) with regenerative generated electrical energy (42, 82, 153) for the production of hydrogen (44, 84, 150),
- a carbon dioxide production plant (Fig 1) for the production carbon dioxide (19, 46, 86) from an ambient air stream (3, 134), wherein the carbon dioxide production plant (Fig. 1) works with an adsorber material that releases bound carbon dioxide again by means of a temperature-vacuum exchange process and
- a methane synthesis (Fig. 2) or Fischer-Tropsch synthesis (Fig. 3) or another hydrocarbon synthesis for synthesising hydrogen (44, 84, 150) and carbon dioxide (19, 46, 86) to methane (57) / gaseous and/or liquid hydrocarbons (114, 115, 116, 117),

whereby heat supply means (4, 10, 11, 12, 13, 14, 128) and/or heat storage means (67) are provided for supplying and/or temporarily storing heat from the synthesis (61, 122) and/or heat from the electrolysis arrangement (155, 156, 157, 158) and/or heat from a combustion device for residual gases (129) from the synthesis process to the carbon dioxide recovery plant (Fig. 1 via 14), wherein the heat (14 via 11) is utilised to carry out the thermal desorption.

**13.** Manufacturing plant according to claim 12,
**characterised in that**
on the methane synthesis reactor (48) or the Fischer-Tropsch synthesis reactor (96) or the reactor of another suitable hydrocarbon synthesis for removing the reaction heat, a steam drum (58, 119) for separating the vapour-containing boiling water (60, 121) leaving the synthesis reactor (48, 96) into boiling water (50, 98) and saturated steam (61, 122) is provided, wherein feed means for the saturated steam (61, 122) to a heat exchanger (14) of a heating medium circuit (11) of the carbon dioxide extraction plant (Fig. 1) are provided.

**14.** Production plant according to one of claims 12 or 13,
**characterised in that**
the electrolysis arrangement (41, 81, 151, 159) is a water vapour electrolysis (151) (SOEC) wherein supply means (155, 156, 157, 158) are provided for transporting heat from a cooling of the formed hydrogen (154)/oxygen stream (150) to a heat exchanger (14) of a heating medium circuit (11) of the carbon dioxide production plant (Fig 1).

**15.** Manufacturing plant according to one of claims 12 to 14,
**characterised in that**

a heat pump arrangement is provided for increasing the temperature of utilisable waste heat from the electrolysis arrangement (155, 156, 157, 158), the synthesis reactor (61, 122) and/or combustion device (129) for residual gases
and/or
storage means (25, 31) are provided for storing and feeding to the methane synthesis (Fig. 2) or Fischer-Tropsch synthesis (Fig. 3) or to another suitable hydrocarbon synthesis of the carbon dioxide (19, 46, 86) continuously produced in the carbon dioxide production plant (Fig. 1).

**Revendications**

**1.** Procédé de production de méthane produit synthétiquement (57)/d'hydrocarbures gazeux et/ou liquides (114, 115, 116, 117),

dans lequel de l'hydrogène (44, 84, 150), issu d'un système d'électrolyse (41, 81, 151) fonctionnant avec de l'énergie électrique produite de façon régénérative, et du dioxyde de carbone (19, 46, 86) sont combinés à cet effet dans une synthèse de méthane (Fig. 2 - 48) ou dans une synthèse de Fischer-Tropsch (Fig. 3 - 96) ou dans une autre synthèse d'hydrocarbures,
**caractérisé en ce que**
le dioxyde de carbone (19, 46, 86) est produit à partir d'un flux d'air ambiant (3, 134) au moyen d'une installation de capture de dioxyde de carbone (Fig. 1), dans lequel le dioxyde de carbone (19, 46, 86) issu du flux d'air ambiant (3, 134) dans l'installation de capture de dioxyde de carbone (Fig. 1) est adsorbé tout d'abord au moyen d'un matériau d'adsorption et est capturé au moyen d'un procédé de transfert température-vide,
dans lequel
la désorption thermique est réalisée pour l'inversion du processus d'adsorption et par conséquent pour la libération du dioxyde de carbone (19, 46, 86) adsorbé, dans lequel de la chaleur (14 par 11) est ainsi introduite dans le matériau d'adsorption (1) et ladite chaleur est extraite au moins partiellement à partir de la synthèse de méthane exotherme (Fig. 2 - 48) ou de la synthèse de Fischer-Tropsch (Fig. 3 - 96) ou d'une autre synthèse d'hydrocarbure appropriée et/ou du système d'électrolyse (41, 81, 151).

**2.** Procédé de production selon la revendication 1,
**caractérisé en ce que**
la chaleur (14) est extraite à partir de vapeur (61, 70, 122) générée au cours du processus de synthèse.

**3.** Procédé de production selon l'une des revendications 1 ou 2,
**caractérisé en ce que**
le système d'électrolyse (41, 81, 151) est une électrolyse de vapeur d'eau (151) fonctionnant avec de l'énergie électrique produite de façon régénérative (SOEC) et de la chaleur générée est extraite à partir du refroidissement (155, 156, 157, 158) d'un flux de gaz d'oxygène et/ou d'hydrogène pour l'inversion (14 par 11) du processus d'adsorption.

**4.** Procédé de production selon l'une des revendications précédentes, **caractérisé en ce que** du gaz résiduel excédentaire et/ou non utilisable (109) généré au cours du procédé de production est brûlé (110) et un gaz de combustion (112) qui se forme est exploité physiquement (130, 134) et/ou énergétiquement (129), dans lequel la chaleur (129) du gaz de combustion est ainsi exploitée pour l'inversion du processus d'adsorption (14 par 11) et/ou le gaz de combustion (112) est employé en tant qu'adjuvant (134) dans le flux d'air ambiant (3).

**5.** Procédé de production selon l'une des revendications 1 à 4, **caractérisé en ce que** de la chaleur résiduelle à basse température est employée à des températures inférieures à 80 °C pour la régénération de l'eau adsorbée dans l'installation de capture de dioxyde de carbone (Fig. 1).

**6.** Procédé de production selon l'une des revendications précédentes, **caractérisé en ce que** un processus de pompage de chaleur est utilisé pour l'augmentation de la température de chaleur issue du processus de synthèse (61, 122) et/ou de l'exploitation de gaz résiduel (129) et/ou du refroidissement dans l'électrolyse (155, 156, 157, 158) et/ou d'une autre chaleur résiduelle de processus.

**7.** Procédé de production selon l'une des revendications précédentes, **caractérisé en ce que**

l'installation de capture de dioxyde de carbone (Fig. 1) fonctionne en continu, dans lequel le besoin en dioxyde de carbone du processus de synthèse (Fig. 2, Fig. 3) est discontinu en raison de variations d'énergie électrique (42, 82, 90, 153) disponible produite de façon régénérative et le dioxyde de carbone (19, 46, 86) capturé est stocké temporairement dans un réservoir d'accumulation (25, 31), dans lequel le stockage temporaire du dioxyde de carbone (19, 46, 86) est effectué de préférence avec une pression plus élevée dans un réservoir de courte durée (25) et un réservoir de longue durée (31) monté en parallèle de celui-ci, dans lequel le stockage temporaire de longue durée est effectué par une liquéfaction du dioxyde de carbone (19, 46, 86).

**8.** Procédé de production selon l'une des revendications précédentes, **caractérisé en ce que** le processus de synthèse (Fig. 2, Fig. 3) est effectué de façon discontinue en raison de variations d'énergie électrique (42, 82, 92, 153) disponible produite de façon régénérative et la chaleur exploitable (61, 122) pour l'installation de capture de dioxyde de carbone (Fig. 1) est stockée temporairement dans un réservoir d'accumulation (67).

**9.** Procédé de production selon l'une des revendications précédentes, **caractérisé en ce que** de l'eau issue de l'atmosphère, capturée pendant le fonctionnement de l'installation de capture de dioxyde de carbone (Fig. 1), est utilisée pour l'électrolyse (41, 81, 151).

**10.** Procédé de production selon l'une des revendications précédentes, **caractérisé en ce que** en plus de l'hydrogène (44, 84, 150), du monoxyde de carbone issu de dioxyde de carbone (19, 46, 86) est également capturé à l'aide du système d'électrolyse (41, 81, 151, 159) fonctionnant avec de l'énergie électrique produite de façon régénérative.

**11.** Procédé de production selon la revendication 10, **caractérisé en ce que** le système d'électrolyse (41, 81, 151, 159) est une électrolyse de vapeur d'eau (151), dans lequel la vapeur est capturée directement à partir du mélange de dioxyde de carbone-vapeur d'eau aspiré pendant la fonction de régénération de l'installation de capture de $CO_2$ à partir du récipient (1), sans que la vapeur d'eau ne soit condensée entre-temps.

**12.** Installation de production pour la fabrication de méthane produit synthétiquement (57)/d'hydrocarbures gazeux et/ou liquides (114, 115, 116, 117), en particulier pour la mise en oeuvre du procédé de production selon l'une des revendications précédentes, comprenant :

- un système d'électrolyse (41, 81, 151) fonctionnant avec de l'énergie électrique (42, 82, 153) produite de façon régénérative pour la production d'hydrogène (44, 84, 150),
- une installation de capture de dioxyde de carbone (Fig. 1) pour la production de dioxyde de carbone (19, 46, 86) à partir d'un flux d'air ambiant (3, 134), dans laquelle l'installation de capture de dioxyde de carbone (Fig. 1) opère avec un matériau d'adsorption, lequel restitue le dioxyde de carbone lié au moyen d'un procédé de transfert température-vide et
- une synthèse de méthane (Fig. 2) ou une synthèse de Fischer-Tropsch (Fig. 3) ou une autre synthèse d'hydrocarbures pour la synthétisation d'hydrogène (44, 84, 150) et de dioxyde de carbone (19, 46, 86) permettant d'obtenir du méthane (57)/des hydrocarbures gazeux et/ou liquides (114, 115, 116, 117),

dans laquelle
il est prévu des moyens d'alimentation de chaleur (4, 10, 11, 12, 13, 14, 128) et/ou des réservoirs thermiques (67) pour l'alimentation et/ou le stockage temporaire de chaleur issue de la synthèse (61, 122) et/ou de chaleur du système d'électrolyse (155, 156, 157, 158) et/ou de chaleur d'un dispositif de combustion pour des gaz résiduels (129) à partir du processus de synthèse vers l'installation de capture de dioxyde de carbone (Fig. 1 par 14), dans laquelle la chaleur (14 par 11) est exploitée pour exécuter la désorption thermique.

13. Installation de production selon la revendication 12,
**caractérisée en ce que**
un tambour à vapeur (58, 119) destiné à la séparation de l'eau bouillante (60, 121) contenant de la vapeur quittant le réacteur de synthèse (48, 96) en eau bouillante (50, 98) et vapeur saturée (61, 122) est prévu sur le réacteur de synthèse de méthane (48) ou le réacteur de synthèse de Fischer-Tropsch (96) ou sur le réacteur d'une autre synthèse d'hydrocarbure appropriée pour l'évacuation de la chaleur de réaction, dans laquelle il est prévu des moyens d'alimentation pour la vapeur saturée (61, 122) vers un échangeur de chaleur (14) d'un circuit d'agent de chauffage (11) de l'installation de capture de dioxyde de carbone (Fig. 1).

14. Installation de production selon l'une des revendications 12 ou 13, **caractérisée en ce que**
le système d'électrolyse (41, 81, 151, 159) est une électrolyse de vapeur d'eau (151) (SOEC), dans laquelle il est prévu des moyens d'alimentation (155, 156, 157, 158) pour le transport de chaleur à partir d'un refroidissement du flux d'hydrogène (154)/d'oxygène (150) formé vers un échangeur de chaleur (14) d'un circuit d'agent de chauffage (11) de l'installation de capture de dioxyde de carbone (Fig. 1).

15. Installation de production selon l'une des revendications 12 à 14, **caractérisée en ce que**

il est prévu un ensemble de pompe à chaleur pour l'augmentation de la température de chaleur résiduelle exploitable issue du système d'électrolyse (155, 156, 157, 158), du réacteur de synthèse (61, 122) et/ou du dispositif de combustion (129) pour des gaz résiduels
et/ou
il est prévu des moyens de stockage (25, 31) pour le stockage et l'alimentation du dioxyde de carbone (19, 46, 86) produit de façon continue dans l'installation de capture de dioxyde de carbone (Fig. 1) vers la synthèse de méthane (Fig. 2) ou la synthèse de Fischer-Tropsch (Fig. 3) ou vers une autre synthèse d'hydrocarbure appropriée.

Fig. 1

Fig. 2

Fig. 3

Fig. 4

EP 3 280 512 B1

**Fig. 5**

Fig. 6

EP 3 280 512 B1

Fig. 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 2049232 A
- EP 1887071 A1
- WO 2008021698 A2
- WO 2006004583 A2
- GB 2448685 A
- US 20140272734 A1
- EP 2638949 A1
- EP 2491998 A1
- WO 2014170184 A1